# EUROPEAN PATENT SPECIFICATION

(11) **EP 1 841 866 B1**
(45) Date of publication and mention of the grant of the patent: **22.04.2009**
(21) Application number: 05854583.1
(22) Date of filing: 19.12.2005
(51) Int. Cl.: C12N 15/11

(54) **NUCLEIC ACIDS FOR APOPTOSIS OF CANCER CELLS**
NUKLEINSÄUREN FÜR DIE APOPTOSE VON KREBSZELLEN
ACIDES NUCLEIQUES POUR L'APOPTOSE DE CELLULES CANCEREUSES

(30) Priority: 25.01.2005 US 646961 P; 08.04.2005 US 669639 P
(43) Date of publication of application: 10.10.2007
(73) Proprietor: Sky Genetics, Inc., Rancho Santa Fe CA 92067 (US)
(72) Inventor: NORTH, Don, Adams, Arlington, Texas 76012 (US)
(74) Representative: Friede, Thomas
(86) International application number: PCT/US2005/045903
(87) International publication number: WO 2006/081008

(56) References cited:
- WO-A-20/06081248

## Description

### FIELD OF THE INVENTION

The present invention, in one embodiment, relates to a nucleic acid having a particular Apoptotic Sequence and able to induce apoptosis in cancer cells while leaving healthy cells unharmed. Other embodiments relate to use of nucleic acids with these sequences for the manufacture of a medicament for the treatment of cancer.

### BACKGROUND

Cancer results when a cell in the body malfunctions and begins to replicate abnormally. These malfunctions result from mutations in the cell's DNA blueprint.

Cancer is treated by attempting to kill cancer cells without harming healthy cells. This relies distinguishing cancer cells from healthy cells, which current methods do quite poorly.

Most DNA cancer research focuses on oncogenes and tumor suppressor genes because these genes have an obvious association with abnormal cell replication. However, these genes are not necessarily the optimum targets for distinguishing between the DNA in cancer cells and the DNA in healthy cells.

### SUMMARY OF THE INVENTION

One embodiment of the present invention relates to a nucleic acid having a sequence of: Seq.ID.No:1, Seq.ID.No:2, Seq.ID.No:3, Seq.ID.No:4, or Seq.ID.No:5, Seq.ID.No:6, Seq.ID.No:7. Another embodiment relates to a composition including a nucleic acid having a sequence of Seq.ID.No:1, Seq.ID.No:2, Seq.ID.No:3, Seq.ID.No:4, or Seq.ID.No:5, Seq.ID.No:6, Seq.ID.No:7. The composition may also include a pharmaceutically acceptable carrier. Yet another embodiment relates to use of nucleic acid having a sequence of: Seq.ID.No:1, Seq.ID.No:2, Seq.ID.No:3, Seq.ID.No:4, or Seq.ID.No:5, Seq. ID No: 7 for the manufacture of a medicament for the treatment of cancer. The cancer cell may be located in a subject with cancer.

### BRIEF DESCRIPTION OF THE DRAWINGS

The present invention may be better understood through reference to the following Figures and Detailed Description.
FIGURE 1 illustrates an Apoptotic Sequence according to an embodiment of the present invention found in the LTBR gene as aligned to the mRNA from healthy cell transcriptomes. The location of a single nucleotide polymorphism (SNP) is indicated.
FIGURE 2 illustrates an Apoptotic Sequence according to an embodiment of the present invention from six different cancer cell lines and four different cancer types, aligned to the corresponding healthy mRNA from 17 different genes.
FIGURE 3 illustrates a method of discovering a candidate Apoptotic Sequence.
FIGURE 4 illustrates the Apoptotic Sequence of Figure 2 in multiple cancer cell lines. PCR conditions for isolation of the sequence are also indicated.
FIGURE 5 illustrates a method for single-priming PCR using an Apoptotic Sequence primer, such as a primer having an Apoptotic Sequence according to an embodiment of the present invention.
FIGURE 6 presents the results of single-priming PCR as analyzed on gels for cDNA from a healthy human and from tumor or blood samples of two cancer subjects, for various candidate Apoptotic Sequence primers. Apoptotic Sequence primers of the present invention include those identified by numbers 5, 8, 9, 11, 14, 60 and 66.
FIGURE 7 diagrams how four Apoptotic Sequence nucleic acids, such as DNA, of the present invention may be combined in an example embodiment to eradicate cancer cells with four unique cancer mutations.

### DETAILED DESCRIPTION

The present invention, in one embodiment relates to nucleic acids having Apoptotic Sequences, which may be able to induce apoptosis in cancer cells while leaving healthy cells unaffected. Other embodiments relate to use of nucleic acids with these sequences for the manufacture of a medicament for the treatment of cancer.

Current cancer research focuses on oncogenes and tumor suppressor genes, which are often mutated in cancer cells, but not in normal cells. However, not all DNA abnormalities associated with cancer are located in an oncogene or a tumor suppressor gene. As a result, candidate Apoptotic Sequences of embodiments of the present invention were located by searching computationally for sequences found in the transcriptome of cancer cells, but generally absent from that of healthy cells. The location of these sequences within the genome was not of primary concern. As a result, some are in tumor suppressor genes or oncogenes, while others are not. However, by not excluding nucleic acid sequences based on their genomic location, unnecessary limitations that may result in a reduction of treatment efficacy are avoided. Further, by excluding sequences normally located in the healthy transcriptome, candidate sequences may have little or no toxicity to normal cells.

To further enhance destruction of cancer cells and possible treatment, sequences located essentially only in cancer cell transcriptomes were further limited to those associated with Apoptotic effects in cells. These sequences are called "Apoptotic Sequences". This is not to say that they are necessarily associated with apoptotic genes, but rather the Apoptotic Sequences themselves, when embodied in a nucleic acid such as DNA can trigger cell death.

By way of example an Apoptotic Sequence may correspond to a DNA mutation that is present in many genes. If expression of all of these genes is simultaneously interfered with, a cell suffocates or starves because of the mass protein deficiency. This is different from programmed cell death normally associated with apoptosis.

Nucleic acids having Apoptotic Sequences of the present invention may be able to induce apoptosis of cancer cells in a variety of manners. First the Apoptotic Sequence nucleic acids may be introduced into the cancer cells by uptake from the environment and/or production within the cell. Next the Apoptotic Sequence nucleic acids may interfere with cellular production of protein, for example by hybridizing with homologous mRNA. This may result in antisense, silencing, or interfering effects, among others. Because the Apoptotic Sequences do not appear in healthy cells, introduction of the Apoptotic Sequence nucleic acids into healthy cells should have little or no effect.

Apoptotic Sequence nucleic acids may include DNA, particularly single-stranded DNA. RNA may also be used, but due to stability and degradation concerns it may be more suitable for production within the cell, for example from a plasmid, than uptake from outside of the cancer cell. Apoptotic Sequence nucleic acids, particularly if introduced from outside of the cancer cell, may be modified, for example by methylation or conjugation with other molecules, to facilitate uptake, transport to areas of the cell where they can be active, or activity in interfering with protein production, among other reasons.

Apoptotic Sequences may also be selected based on routine and repetitive occurrence in cancer RNA transcripts, particularly transcripts from different genes, as opposed to single occurrences in one RNA transcript. This selects for Apoptotic Sequences that can identify a common mutation in multiple genes. Further, function of such an Apoptotic Sequence may not be dependent on the expression level of a single gene, but may instead benefit from multiple expression levels. This may allow Apoptotic Sequences to affect a wide variety of cancer cells. Coupled with the low or non-existent level of harm to normal cells, this may allow identification and specific destruction of cancer cells even in samples having relatively low numbers of cancer cells, such as metastasized cells in blood.

Further, the repetitive occurrence of Apoptotic Sequences in multiple genes may allow the simultaneous disruption of protein production from these genes. For example, cancer cell death may result from ribosomal protein deficiency.

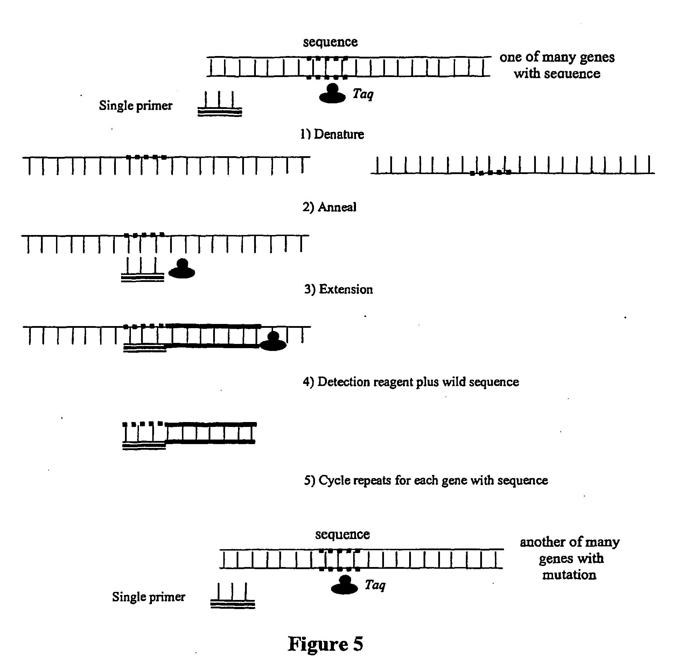

In the same manner that the Apoptotic Sequences repetitively occur in multiple genes, they also repetitively occur in multiple cancer types. The Apoptotic Sequences are not cancer type specific, although each one may have a higher presence in a single cancer type, and/or in one individual subject over another. As a result, it may be desirable to develop a cancer profile for a subject or sample prior to attempting destruction of cancer cells, such as by treatment. This profiling is easily facilitated using a 20 ml blood sample and the Apoptotic Sequences as RT-PCR primers. One method of using Apoptic Sequences as primers is shown in FIGURE 5. Biopsies and other samples may be used, but are not normally required. For example, the presence of Apoptotic Sequences may be detected in the metastasized cancer cells of a subject's blood, which then assures their presence in the subject's tumors.

Example Apoptotic Sequences of the present invention are shown in *Table 1.* The ID number indicated in the table is used when referring to these sequences throughout this specification, for example in the experiments described in the Figures. Although Apoptotic Sequences need not all be a specific length, the Apoptotic Sequences of Table 1 are all 17 base pairs in length, allowing specificity, but facilitating function.

**Table 1: Apoptotic Sequences**

| **ID** | **Apoptotic Sequence** | |
|---|---|---|
| 5 | AAGGGGGTTCCTTGGGC | (Seq.ID.No:1) |
| 8 | CCTGAGCAAACCTGAGC | (Seq.ID.No:6) |
| 9 | GGCCTGCCAGAAGCACA | (Seq.ID.No:2) |
| 11 | CGCATGCGTGGCCACCA | (Seq.ID.No:7) |
| 14 | GCCGATTAACACCAGCC | (Seq.ID.No:3) |
| 60 | CGATTAACCACCGGCCT | (Seq.ID.No:4) |
| 66 | TTGAACCCTAGGCATGT | (Seq.ID.No:5) |

In a particular embodiment, one or more Apoptotic Sequences may provided in a nucleic acid, such as DNA, and be used to induce apoptosis in a cancer cell. An Apoptotic Sequence nucleic acid may be provided in a physiologically acceptable carrier, such as PNAS or PBS or CSF solution, to form an Apoptotic Sequence serum. This serum may be administered to the cancer cell. For example, it may be administered directly to the blood or spine. A normal dosage, based on body weight, of each Apoptotic Sequence DNA from Table 1 has been administered to several mice, and 10 times the normal dosage has been administered to 5 mice. Normal DNA administration was 5 mg per 1 kg of body weight, mixed in a ratio of 10 mg DNA per 1 ml PBS or CSF.

### Multi-Gene Aspect

Many genes may be associated with each Apoptotic Sequence. Sometimes, hundreds of mRNA transcripts may contain a single Apoptotic Sequence. The common appearance of these Apoptotic Sequences, which may be cancerous mutations, in many genes is not presently understood. However, it is this commonality in multiple genes that may facilitate the cancer cell-differentiating ability of the Apoptotic Sequences and their apoptosis ability.

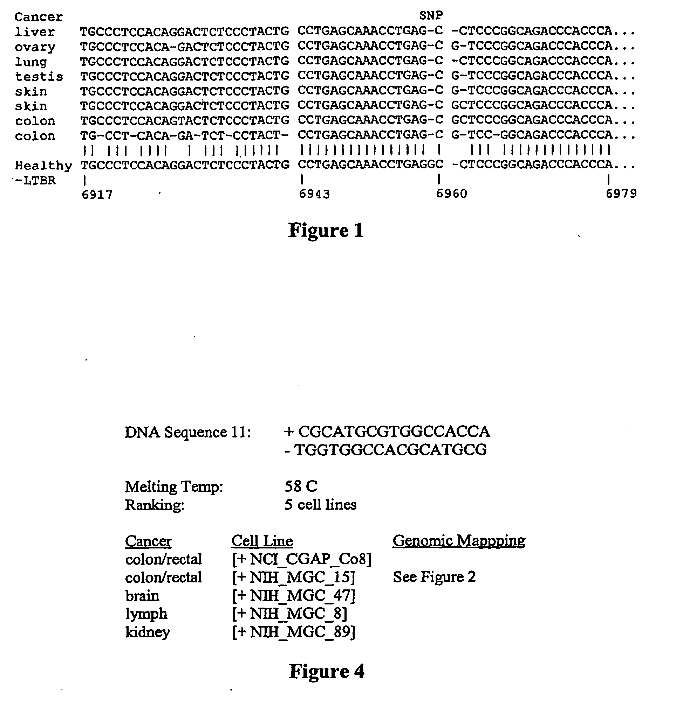

While most of the candidate Apoptotic Sequences are located in genes with no currently known relevance to cancer, some are located in genes known to be important in cancer. These sequences often manifest themselves as SNPs, cryptic splicing and other genetic defects. For example, FIGURE 1 illustrates an Apoptotic Sequence found in the Lymphotoxin Beta Receptor (LTBR) gene.

FIGURE 1 shows that the same point mutation occurs in the same gene in different subjects with different types of cancer. Specifically, FIGURE 1 shows a portion of an alignment between LTBR mRNA from eight different cancer cell lines and six different cancer types, mapped to the corresponding healthy LTBR mRNA. As the figure shows, the eight cancer LTBRs vary slightly between each other and the healthy LTBR. However at location 6959 bp, the cancer LTBRs vary identically, each missing a guanine (G) and yielding the same Apoptotic Sequence, CCTGAGCAAACCTGAGC (Seq.ID.No:6).

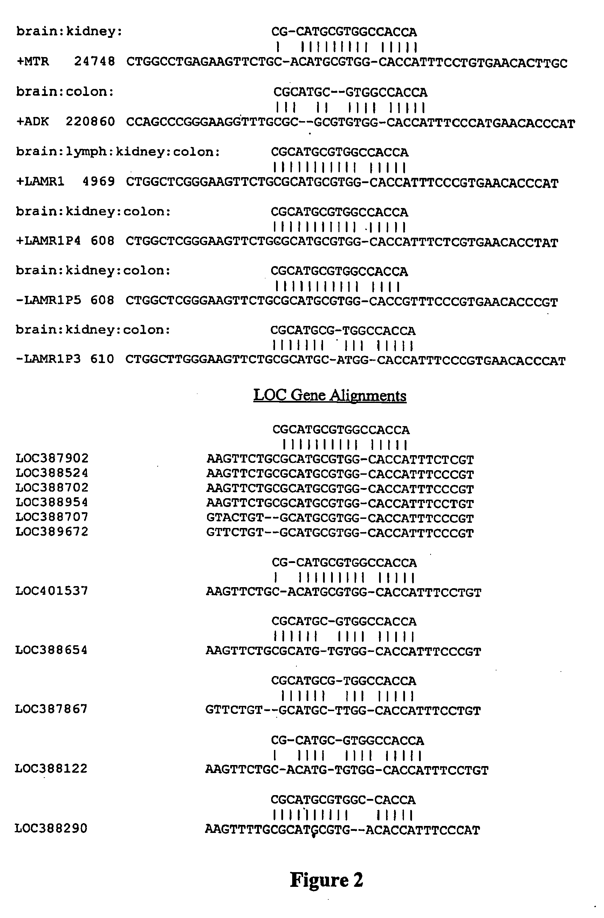

FIGURE 2 shows that the same Apoptotic Sequence can result from common regions in different mutations, different genes, in different subjects, and different types of cancer. Specifically, FIGURE 2 shows a portion of an alignment between mRNA from four different cancer cell lines and four different cancer types, aligned with the corresponding healthy mRNA from different genes. The overall alignments vary from gene to gene, but each has a common region yielding the Apoptotic Sequence, CGCATGCGTGGCCACCA (Seq.ID.No:7).

The Apoptotic Sequences shown in FIGUREs 1 and 2 are not dependent on any common functionality among the genes in which they appear, or in the tissues in which these genes are expressed. Further, none of the sequences has been found in the healthy human transcriptome. Therefore the presence of these sequences in any mRNA transcript, not just those from genes shown in the figures, may be an indicator of cancer's presence in the host cell.

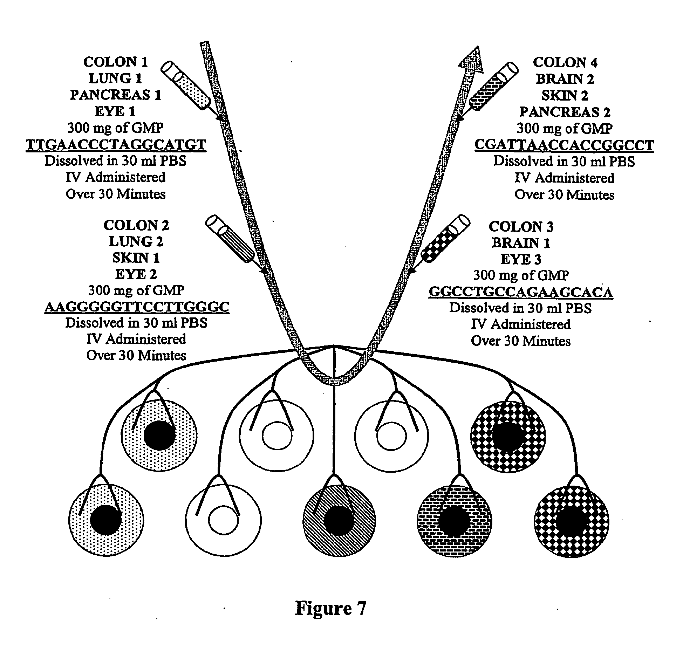

Apoptotic Sequences may be common to many genes and many cancers. This does not mean that they will exist in every cancer cell line or cancer subject. Therefore it is desirous to know which Apoptotic Sequences correspond to a subject's individual cancer. Then the sequences can be used to make an appropriate Apoptotic Sequence serum. This is illustrated in FIGURE 7 where a single cancer may require multiple serums to eradicate all the cancer cells. The figure also shows the overlap in the Apoptotic Sequences between cancer types. So one serum may be effective against many types of cancer, but no two cancer subjects should be presumed as having the same cancer mutations. This flexibility gives the Apoptotic Sequences' serums superiority over the rigid targeting of current chemotherapies.

### Computational Identification of Candidate Apoptotic Sequences

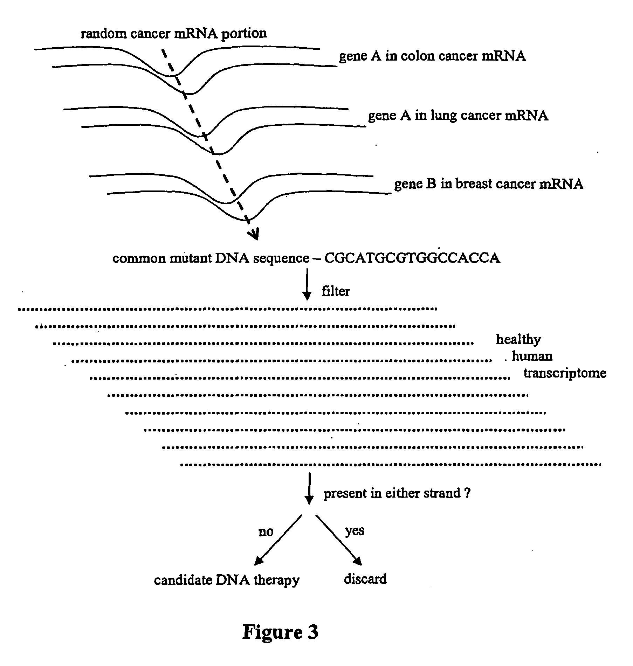

The Apoptotic Sequences of the present invention were isolated using proprietary software and information from public databases by recording genetic information about cancerous and healthy cells and tissues. Specifically, using proprietary software and supercomputers, random portions of mRNA data from cancer cell lines were compared to all the available mRNA data from all healthy cell lines, as diagramed in FIGURE 3. After candidate Apoptotic Sequences are computationally identified, they are tested in-vitro for cancer cell differentiation and apoptosis effects.

The computational analysis yields a set of candidate Apoptotic Sequences for various cancer types by the screening method illustrated in FIGURE 3. One type off cancer may be given priority over other types by requesting that the computers only show candidates that are known to have occurred at least once in the priority cancer type. This is determined by the cell line or cDNA library name. One example showing this information is provided in FIGURE 4. TABLE 6 shows the top candidates when colon cancer is the priority cancer type. The public databases are robust enough to allow such a table to be constructed for any cancer type. TABLE 6 also shows the various cancers known to have contained the sequences, and the multiple genes known to contain the sequences.

### Multi-Gene or Single Gene Apoptotic Sequences

As mentioned earlier, the multi-gene aspect of some Apoptotic Sequences facilitates cancer cell death through protein deficiency. However, there are also cases where the Apoptotic Sequences appear in a single gene, and knocking out this gene triggers cell death. In such cases, the cancer mutations in these genes may be providing a means to identify and differentiate cancer cells, i.e. acting like oncogenes, and providing a mechanism to force apoptosis on the cancer cells, i.e. acting like apoptotic genes. This duality of therapeutic importance in a single gene is highly uncommon, but may identify Apoptotic Sequences particular useful in inducing death of some cancer cells.

TABLE 2 shows further information for the Apoptotic Sequences of TABLE 1. In particular, it provides their multi-gene or single gene mapping characterizations. In the case of single gene Apoptotic Sequences, the recognized National Institutes of Health (NIH) gene names are provided. Also provided and shown in parentheses are common alias names given to the mapped gene, and genes that are similar to the mapped gene and contain the Apoptotic Sequence as well. In the latter case, most of these genes are predicted and have yet to be characterized by NIH.

**Table 2: Gene Mapping of Apoptotic Sequences**

| **ID** | **Phosphorothioated Apoptotic Sequence** | **Healthy cells** | **Subject R colon cancer cells** | **Gene Characterization** | **(Alias Names) & Similar Genes** |
|---|---|---|---|---|---|
| 5 | AAGGGGGTTCCTTGGGC | 10% | 82% | multi-gene | |
| 9 | GGCCTGCCAGAAGCACA | 9% | 70% | GNB2L1 | (RACK1) |
| 14 | GCCGATTAACACCAGCC | 15% | 72% | multi-gene | |
| 60 | CGATTAACCACCGGCCT | 12% | 73% | multi-gene | |
| 66 | TTGAACCCTAGGCATGT | 8% | 83% | EEF1A1 | EEF1A2 |
| | | | | | LOC441032 |
| | | | | | LOC440595 |
| | | | | | LOC442709 |
| | | | | | LOC442332 |

### In-vitro Cancer Cell Differentiation Lab Tests

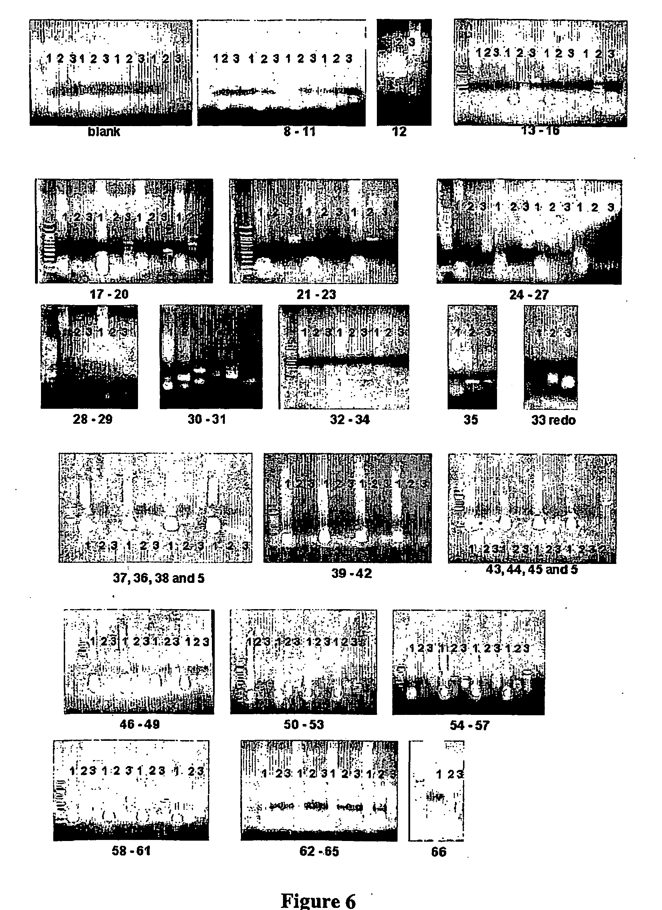

The cancer cell differentiation abilities of the candidate Apoptotic Sequences from TABLE 6 were tested for their presence in cancer cells and absence in healthy cells. The general method of this testing is shown in FIGURE 5. Testing was conducted using an excised 9 mm tumor and a 20 ml blood sample, taken at different times, from Subject R and a 20 ml blood sample from Subject H. Subject R was a female human patient with metastasized colon cancer. Subject H was a male human patient also with metastasized colon cancer. The multi-gene, one-to-many aspect of Apoptotic Sequences yields sensitivity sufficient to allow detection of metastasized cancer cells even in blood samples in addition to biopsies, as shown in FIGURE 6. The healthy control sample used in the tests must be carefully selected because of this sensitivity. It is possible for cancer to be detected in what is otherwise believed to be healthy cells. Therefore, a healthy control sample from tissue not normally associated with cancer, like vascular walls, may be used.

TABLE 3 shows the results of single priming RT-PCR using the primers with the Apoptotic Sequences from TABLE 6, the three cancer samples, and a vascular wall healthy control sample. A plus sign in TABLE 3 indicates a sequence's presence and a minus sign indicates a sequence's absence. Those sequences found in the healthy control sample were discarded from the candidate Apoptotic Sequence pool, while the others are available for subsequent cell death tests.

**Table 3: Candidate Apoptotic Sequence RT-PCR Detection Tests**

| **Candidate Apoptotic Sequence Number** | **Healthy cDNA** | **Human colon cancer tumor cDNA from Subject R** | **Human colon cancer blood cDNA from Subject R** | **Human colon cancer blood cDNA from Subject H** |
|---|---|---|---|---|
| 1 | + | + | + | + |
| 2 | - | - | - | - |
| 3 | - | - | - | - |
| 4 | - | - | - | - |
| 5 | - | + | + | + |
| 6 | - | - | - | - |
| 7 | - | - | - | - |
| 8 | - | + | + | + |
| 9 | - | + | + | + |
| 10 | - | - | - | - |
| 11 | - | + | + | + |
| 12 | - | + | + | - |
| 13 | - | - | + | - |
| 14 | - | + | + | + |
| 15 | - | - | - | - |
| 16 | - | + | + | + |
| 17 | - | - | + | - |
| 18 | - | - | - | |
| 19 | - | - | - | + |
| 20 | - | | + | - |
| 21 | - | - | - | + |
| 22 | - | - | + | - |
| 23 | - | + | - | - |
| 24 | - | - | - | - |
| 25 | - | - | + | + |
| 26 | - | + | + | + |
| 27 | - | - | - | - |
| 28 | - | - | + | - |
| 29 | - | - | - | - |
| 30 | - | + | + | + |
| 31 | - | + | + | - |
| 32 | - | - | + | + |
| 33 | - | + | + | + |
| 34 | - | - | - | - |
| 35 | - | + | + | + |
| 36 | - | - | - | - |
| 37 | - | - | + | + |
| 38 | - | - | - | + |
| 39 | - | - | - | - |
| 40 | - | - | - | - |
| 41 | - | - | - | - |
| 42 | - | - | + | - |
| 43 | - | - | - | - |
| 44 | - | - | - | - |
| 45 | - | - | - | - |
| 46 | - | - | - | - |
| 47 | - | - | - | - |
| 48 | - | - | + | + |
| 49 | - | - | - | - |
| 50 | - | - | - | - |
| 51 | - | - | + | + |
| 52 | - | - | - | - |
| 53 | - | + | + | + |
| 54 | - | - | - | + |
| 55 | - | + | + | + |
| 56 | - | - | + | + |
| 57 | - | + | + | + |
| 58 | + | - | + | - |
| 59 | - | - | + | + |
| 60 | - | + | + | + |
| 61 | - | - | + | + |
| 62 | - | - | + | - |
| 63 | - | - | - | + |
| 64 | - | - | + | - |
| 65 | - | - | - | + |
| 66 | - | + | + | + |

### In-Vitro Cancer Cell Death Tests

Once a candidate Apoptotic Sequence has shown an ability to differentiate between healthy and cancer cells, one may then establish its ability to kill the cancer cells. A sequence's ability to differentiate between healthy and cancer cells does not necessarily mean it can kill the cancer cells. Although most of the candidate Apoptotic Sequences can be used to knock-out or otherwise interfere with expression of many genes in cancer cells, this may not be sufficient to kill the cells. Therefore there may be significant attrition between the number of sequences that can differentiate only and become a candidate Apoptotic Sequence, and the number of sequences that can differentiate and kill cancer cells and are thus truly Apoptotic Sequences.

Twenty candidate Apoptotic Sequences in TABLE 3 were selected for subsequent cell death tests. The selected Apoptotic Sequences were embodied in phosphorothioated DNA and enclosed in commercially available lipids, the lipids being a standard transfection technique for in-vitro anti-sense DNA tests. The resulting Apoptotic Sequence sera were applied to cell cultures grown from a tumor removed from Subject R. TABLE 4 shows the results, including healthy and cancer cell death percentages. Blanks indicate results in which substantial amounts of both healthy cells and an cancer cells were killed.

**Table 4: Candidate Apoptotic Sequence Cell Death Tests**

| **ID** | **Phosphorothioated Apoptotic Sequence** | **Healthy cells** | **Subject R colon cancer cells** |
|---|---|---|---|
| 5 | AAGGGGGTTCCTTGGGC | 10% | 82% |
| 8 | GCTCAGGTTTGCTCAGG | 28% | 46% |
| 9 | GGCCTGCCAGAAGCACA | 9% | 70% |
| 10 | CCAACTGGATCCCAGGT | | |
| 11 | TGGTGGCCACGCATGCG | 20% | 75% |
| 12 | CGGATGTCCCTGCTGGG | | |
| 14 | GCCGATTAACACCAGCC | 15% | 72% |
| 16 | GCCGATTCACACCCAGC | | |
| 20 | GCCTCGTACCTAGCCG | | |
| 23 | CGCCTCGGCCGATTAAC | | |
| 26 | GGCCGATTTACACCCGG | | |
| 30 | TCGGCCGATTAACCCCA | | |
| 31 | CCGATTAACACCGGCCT | | |
| 33 | GCTGTTGTCATACTTGCT | | |
| 35 | CCACGTGATGTAGACTG | | |
| 53 | CCCAGCCTCGTACCTAG | | |
| 55 | CAGCCTCTACCTAGCCTT | | |
| 57 | CACCGGCCTCGTACCT | | |
| 60 | CGATTAACCACCGGCCT | 12% | 73% |
| 66 | TTGAACCCTAGGCATGT | 8% | 83% |

TABLE 5 shows the final Apoptotic Sequences taken from TABLE 4 based on lowest healthy cell death percentages. Although all of the sequences causing cancer cell death in TABLE 3 also showed evidence of causing some healthy cell death, it is difficult to determine low cell death percentages such as those shown in the table.

**Table 5 Death Rate of Healthy and Colon Cancer Cells When Exposed to Apoptic Sequence DNA**

| **ID** | **Phosphorothioated Apoptotic Sequence** | **Healthy cells** | **Subject R colon cancer cells** |
|---|---|---|---|
| **5** | **AAGGGGGTTCCTTGGGC** | **10%** | **82%** |
| **9** | **GGCCTGCCAGAAGCACA** | **9%** | **70%** |
| **14** | **GCCGATTAACACCAGCC** | **15 %** | **72%** |
| **60** | **CGATTAACCACCGGCCT** | **12%** | **73%** |
| **66** | **TTGAACCCTAGGCATGT** | **8%** | **83%** |

### In-Vivo Mice Tests

Because healthy cell death is a direct reflection of toxicity, fifteen mice were given different Apoptotic Sequence sera doses made from short single strand DNA having the Apoptotic Sequences in TABLEs 1 and 5. Three mice were given single doses of each serum based on 5 mg DNA per 1 kg of body weight. This came to about 0.2 mg DNA for each mouse.

After several weeks with no apparent changes in mice behavior, five mice were given doses of each sera based on 50 mg DNA per 1 kg of body weight. This came to about 2 mg DNA for each mouse. Again, after several weeks no apparent changes in mice behavior were observed.

Thus it appears safe to administer doses of Apoptic Sequence sera between approximately 5 mg - 50 mg DNA per 1 kg of body weight. Dosage may also be limited to no more than approximately 25 mg DNA per 1 kg body weight. For such sera, the last test five mice were given the equivalent of a 10x dose without any side effects. Because mice are standard toxicity model for humans, these dosages may be appropriate for administration to a human as well.

### Apoptotic Sequence Sera

Although every Apoptotic Sequence in TABLEs 1 and 5 showed no apparent side effects or toxicity in the mice, FIGURE 7 shows the four remaining Apoptotic Sequence sera. Each of these sera may be administered to a cancer cell, including a cancer cell in a human subject, singly or in conjunction with one or more additional sera. When applied to a human subject, they may also be combined with other therapeutics, such as chemotherapeutics and radiotherapeutics, or other treatments, such as surgery to remove a tumor, or injection into a tumor or its blood supply, or in proximity to a tumor. The sera of FIGURE 7 may be applied to any type of cancer cell, but they may show increased effectiveness in inducing death of colon cancer cells because they were initially identified in a screen to preferentially select colon cancer candidate Apoptotic Sequences.

A typical low dose of an Apoptotic Sequence serum for an average human may include about 300 mg of phosphorothioated DNA, and a high dose may include about 1500 mg. The serum may be administered weekly. It may include multipel Apoptotic Sequence nucleic acids. Administration may continue until no further signs of cancer are detected and may be resumed in cancer signs reappear. Tumor markers, such as those corresponding to the Apoptic Sequences maybe measured after each administration and administered sera may be adjusted as a result.

One example of complete a administration formula and protocol for administration of one or more Apoptotic Sequence serum to one human subject may include the following steps. First, approximately 300 mg cGMP Phosphothioated DNA having an Apoptic Sequence may be ordered from any commercial source or prepared. It may be desalted or HPLC purified. The Phosphorothioated DNA is quite stable when stored at -20°C in the lyophilized form. It is stable for one week when stored at 4°C. Second, sterile PBS (phosphate buffered saline) or artificial CSF (cerebrospinal fluid) may be provided. Third, the 300 mg of Phosphorothioated DNA may be prepared with 30 ml of sterile PBS or artificial CSF to form an Apoptotic Sequence serum. These may be mixed by shaking gently on a nutator at 4°C or gently pipetting up and down at 4°C. Finally, the Apoptotic Sequence serum may be administered to a subject by slow IV drip for 30 minutes.

Each Apoptic Sequence serum should no longer be detectable in the body after 48 hours. Effects on cancer cells may be detectable within 24 hours of administration.

The Apoptotic Sequence sera have little to no effects on healthy tissues, such as liver toxicity.

### Apoptotic Cancer Serum Speed and Cost

Although multiple doses of one or more Apoptic Sequence sera may be used to kill cancer cells, in some subjects as little as a single dose may be effective to induce cancer remission. In any event, the costs associated with Apoptotic Sequence sera of the present invention may be low as compared to convention cancer treatments. For example, using the administration protocol described above, 300 mg of cGMP Phosphorothioated DNA having an Apoptic Sequence 17 bases long may be obtained in its desalted form for $3500, with at least 90% purity through HPLC for $4000, and with at least 95% purity through HPLC for $4500. Sufficient PBS or CSF for several doses may be obtained for approximately $200. This a single dose of an Apoptic Sequence sera may cost as little as between $3500 - $4700.

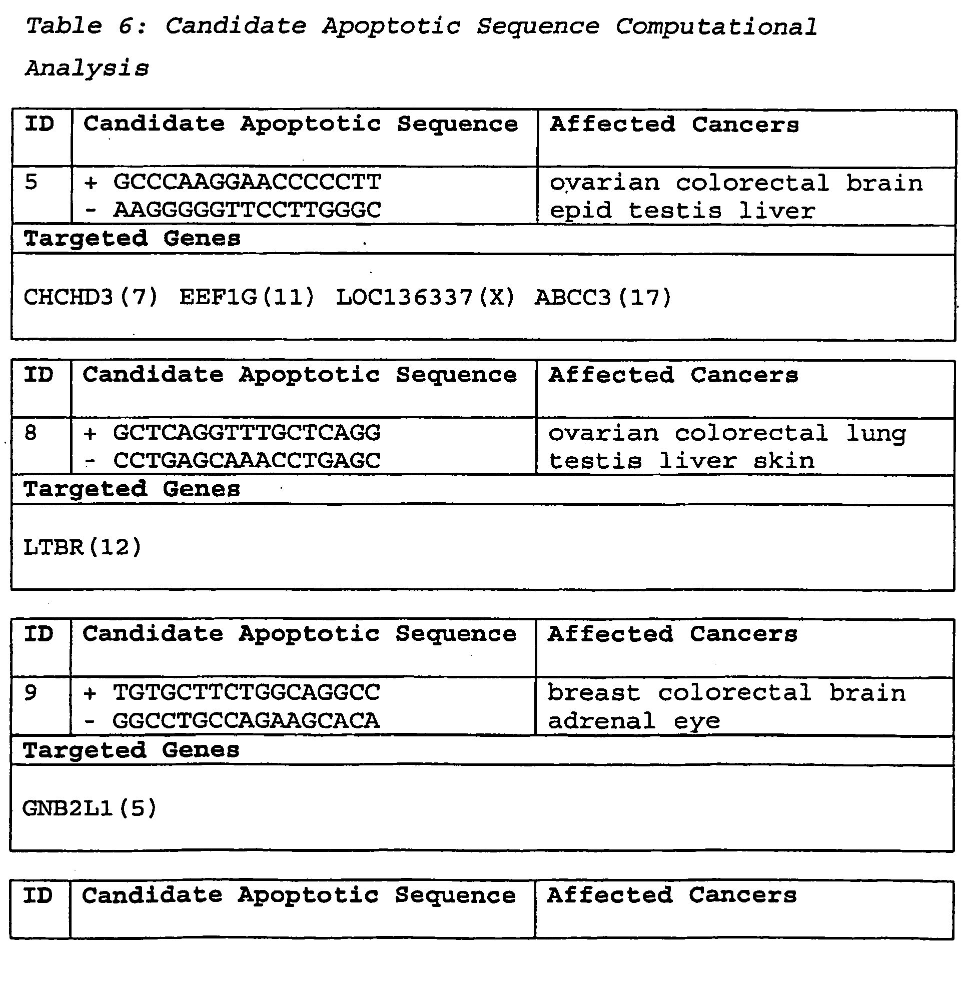

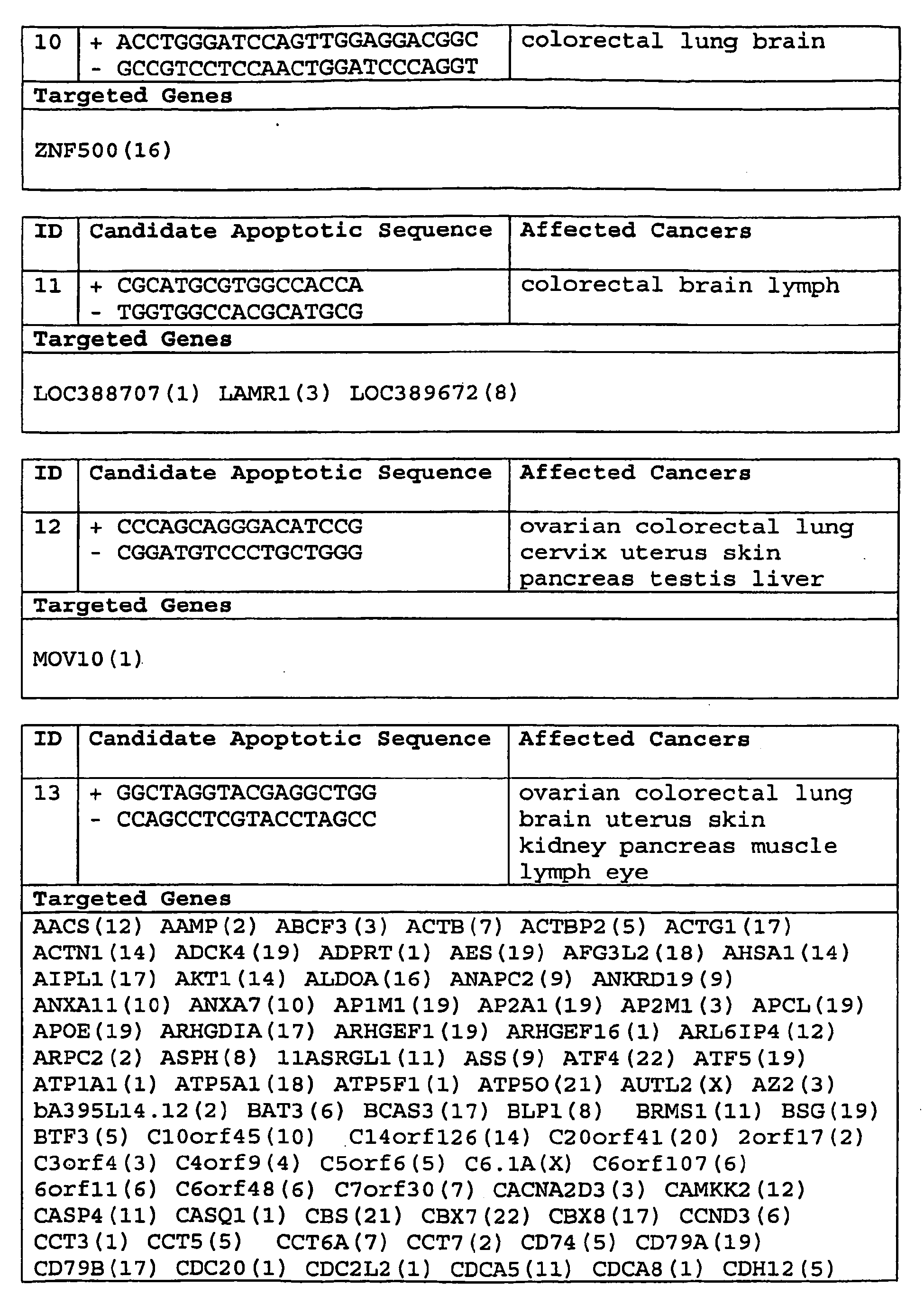

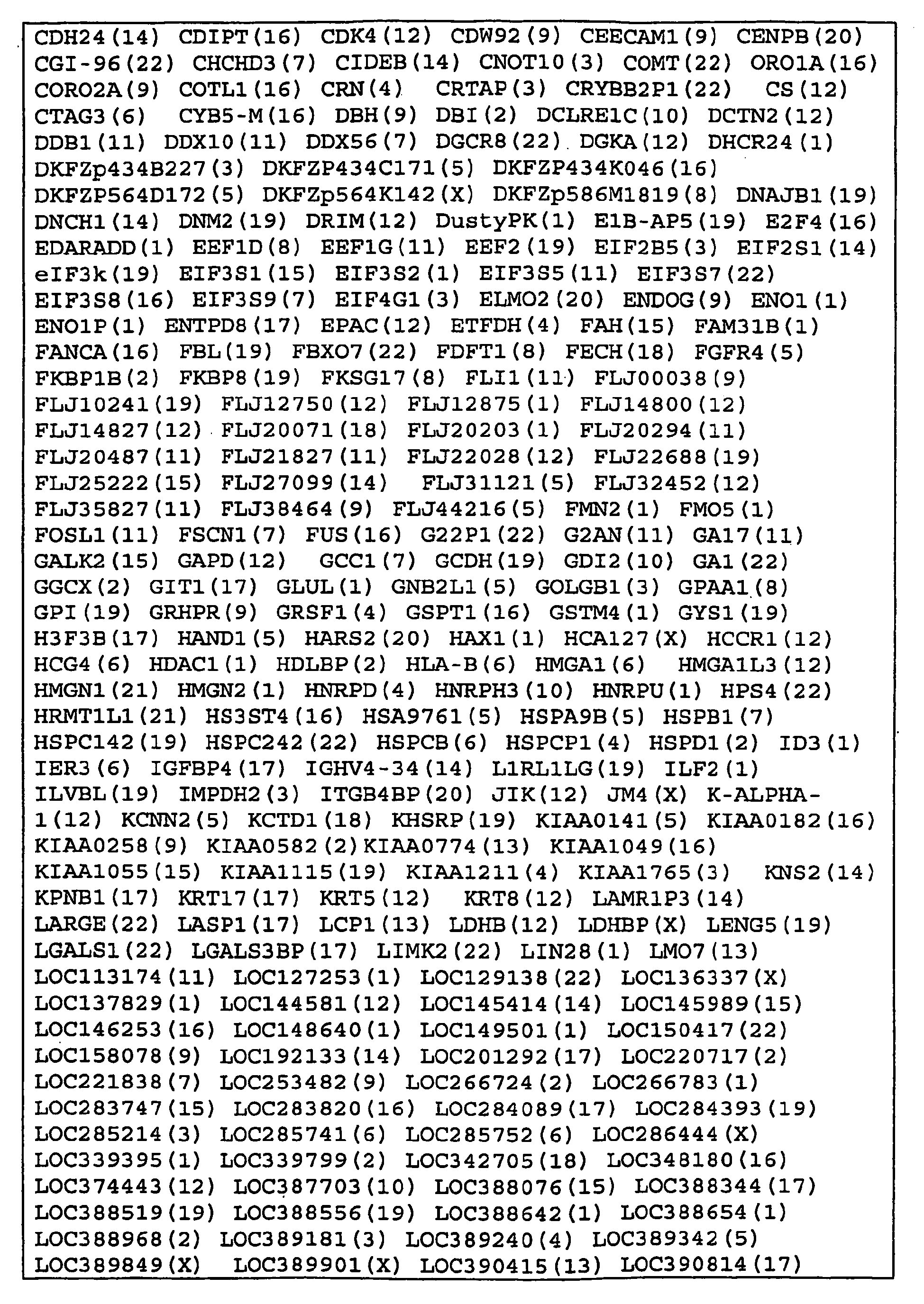

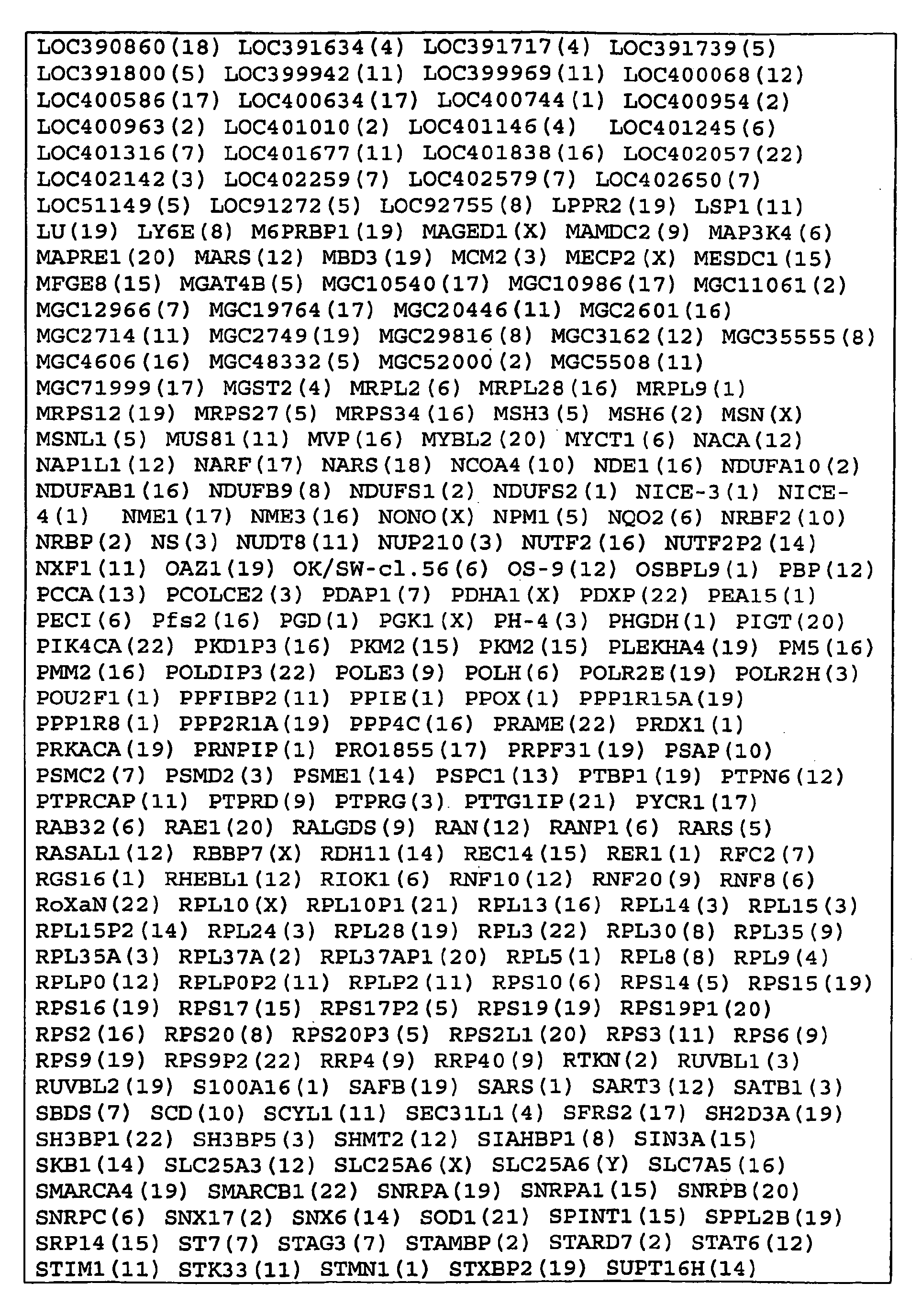

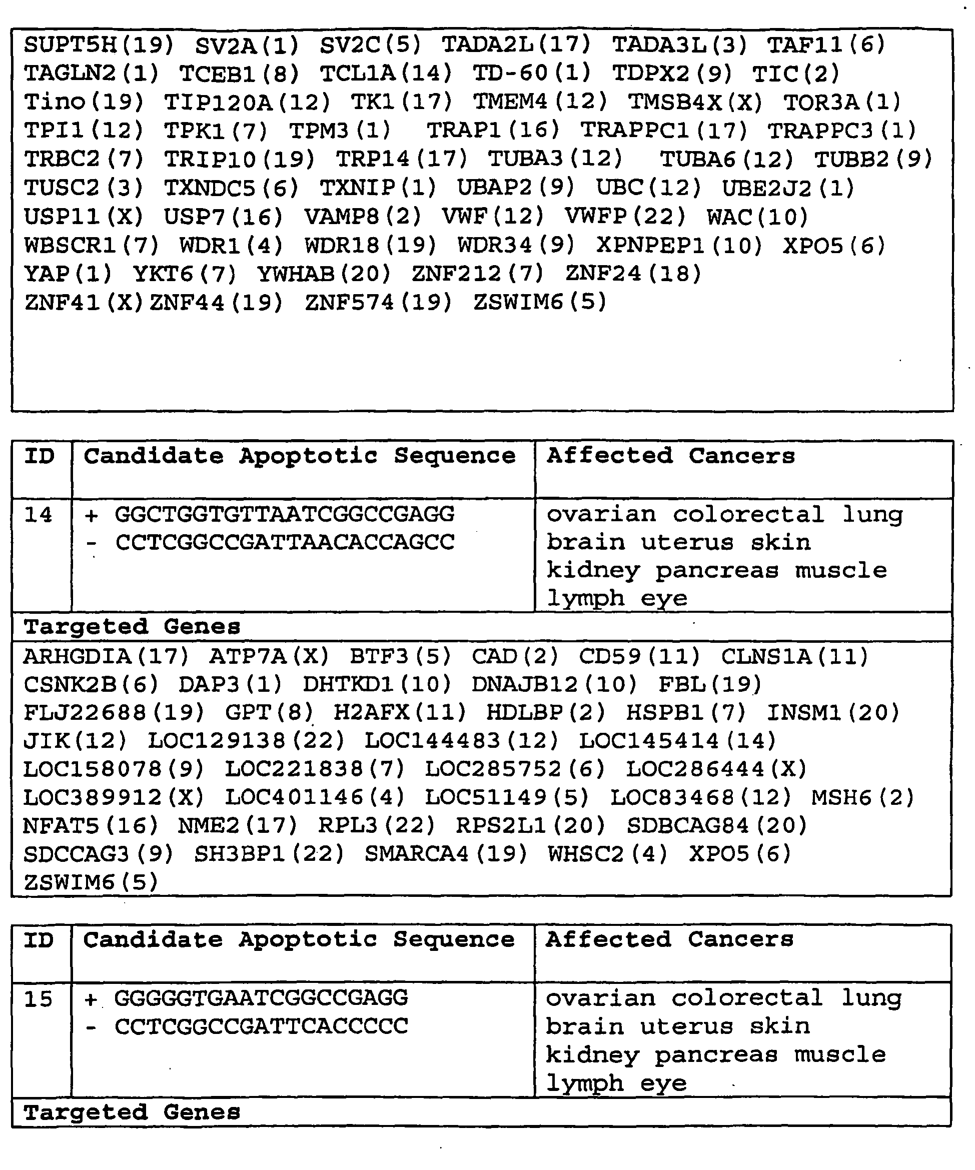

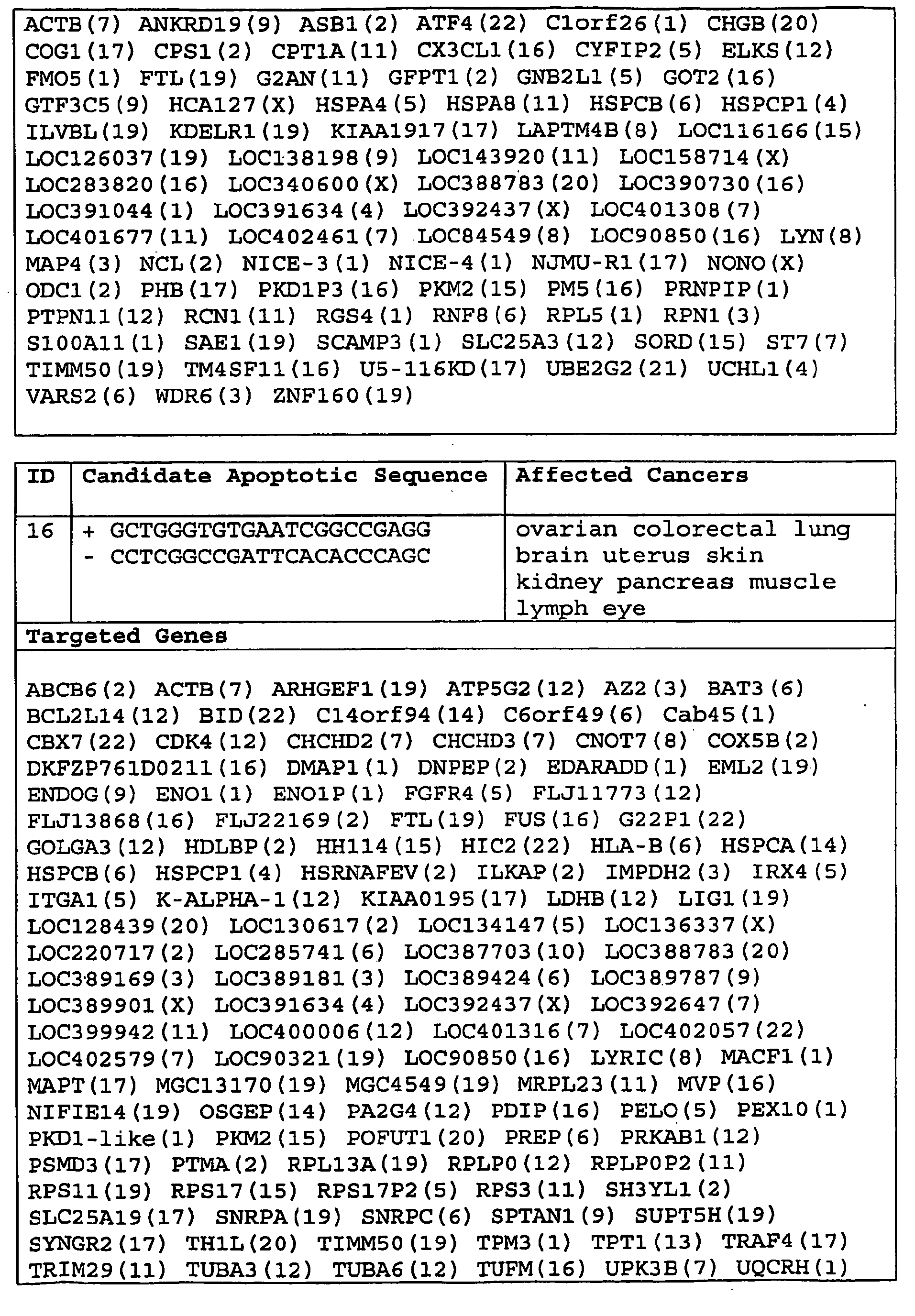

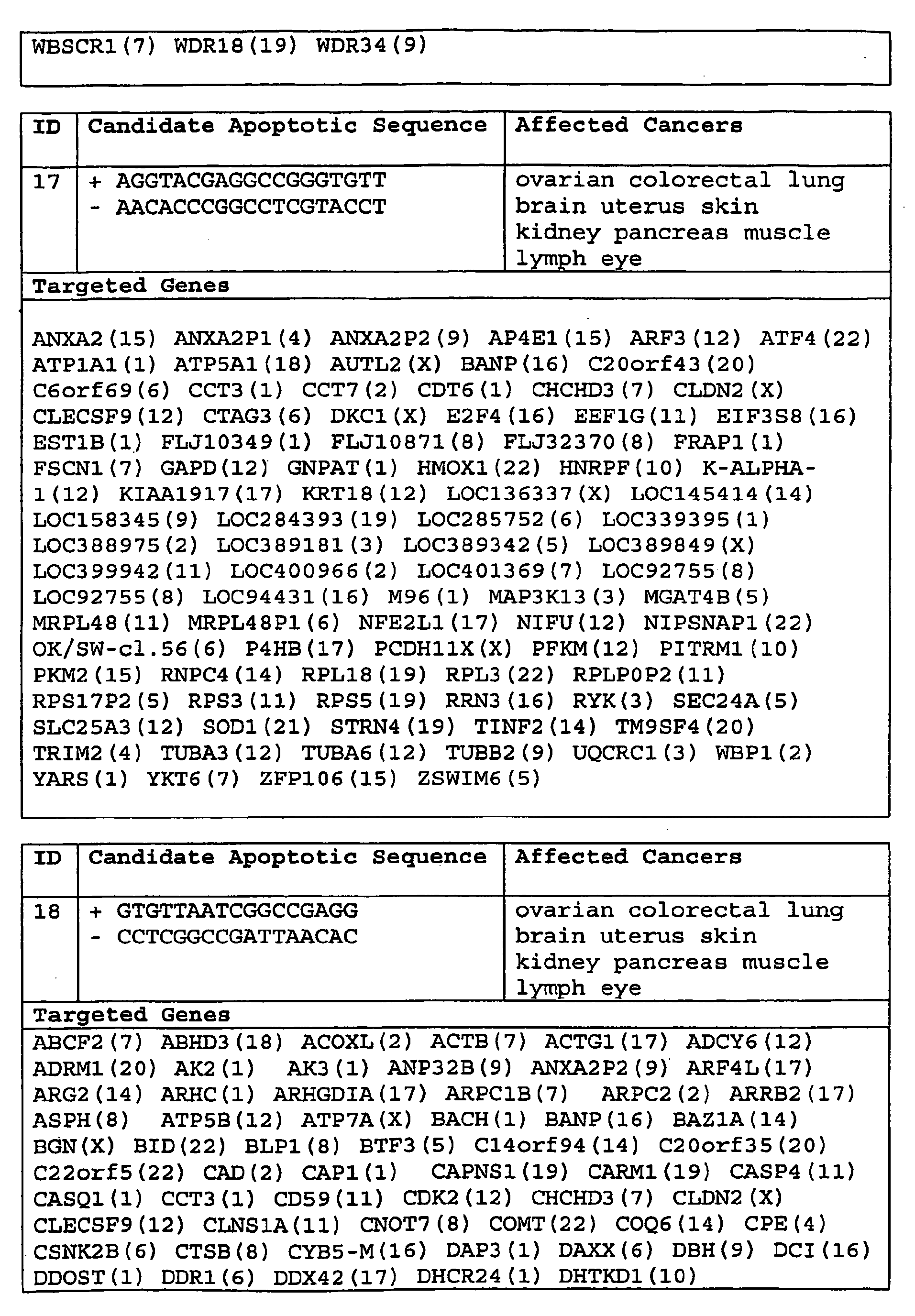

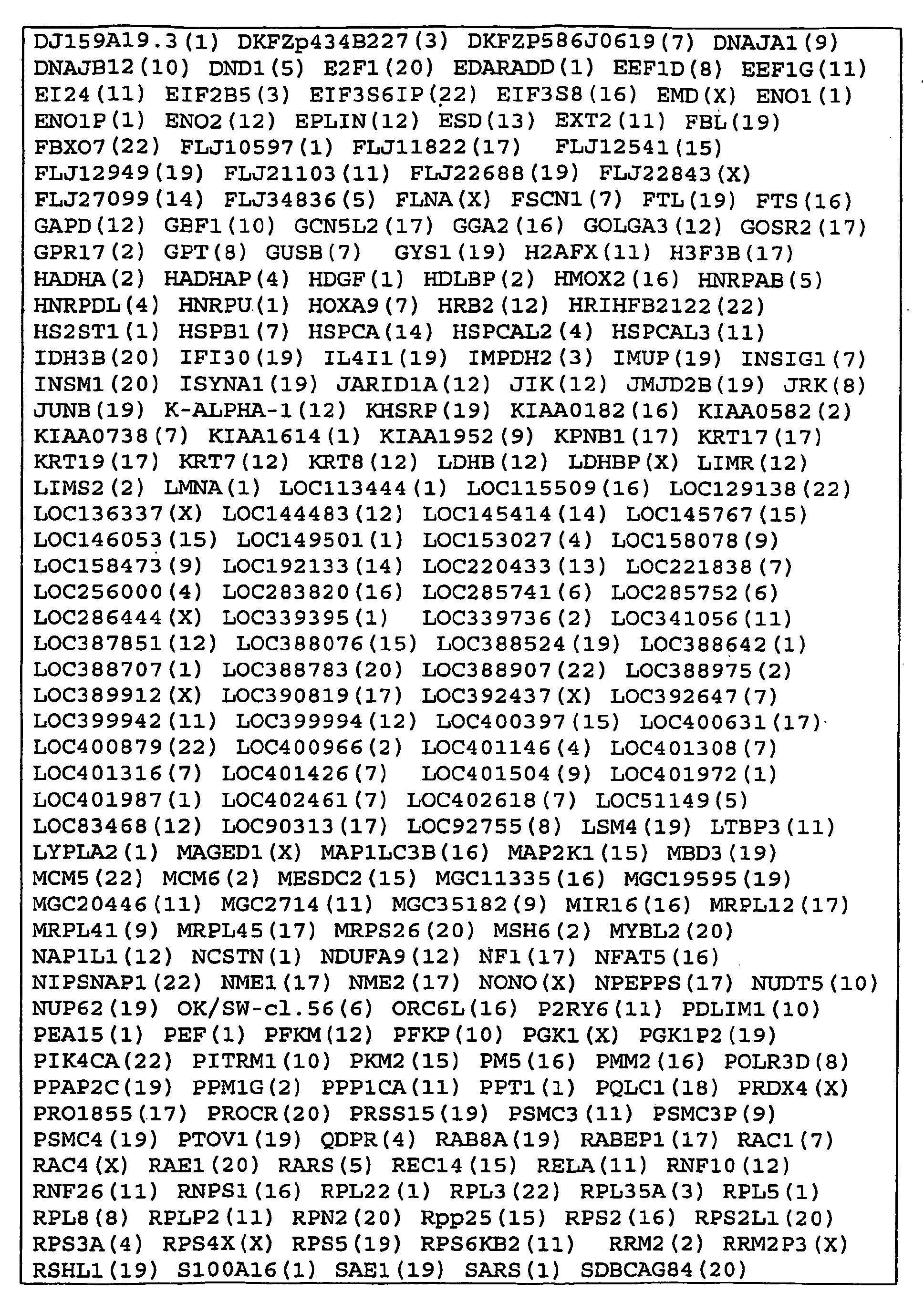

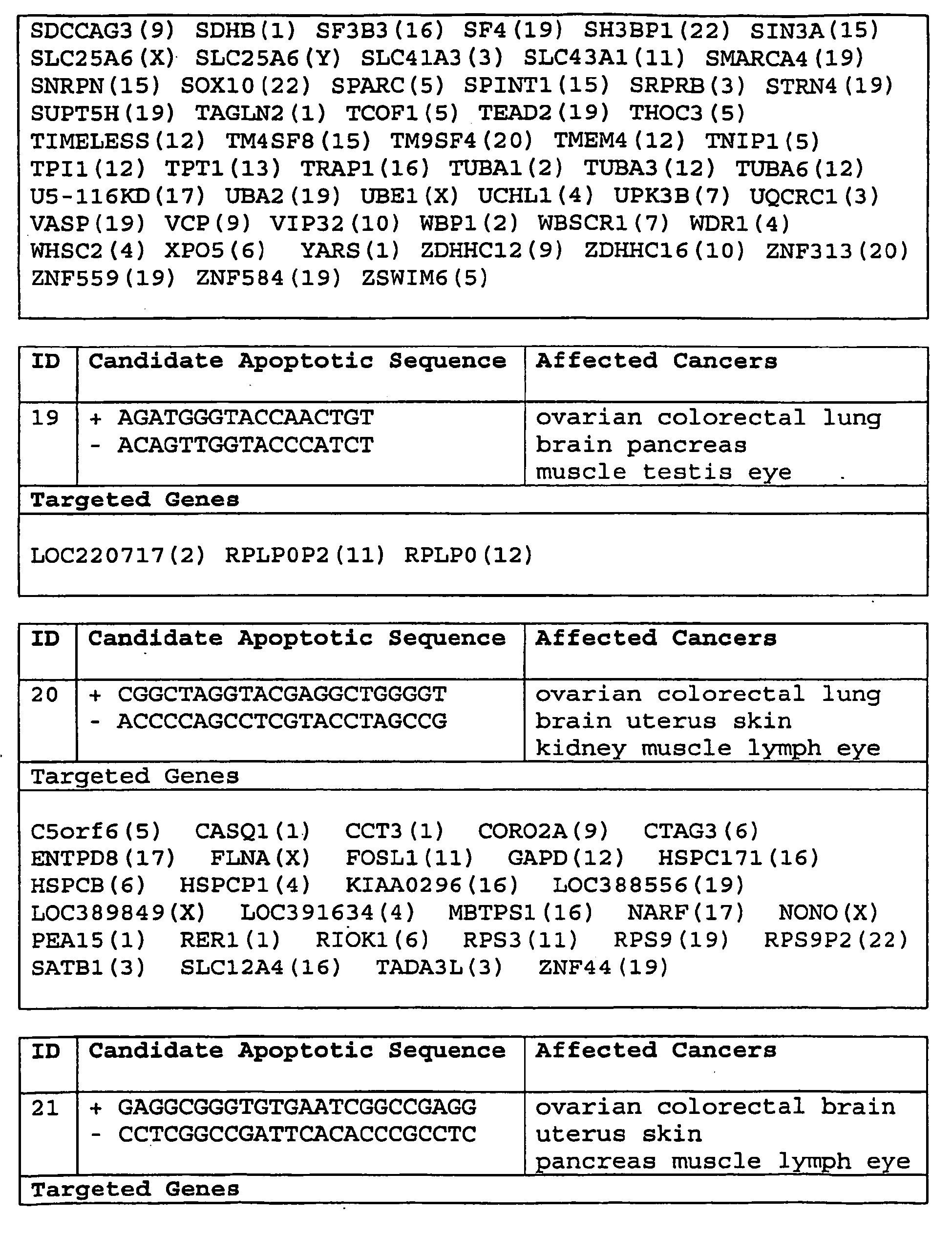

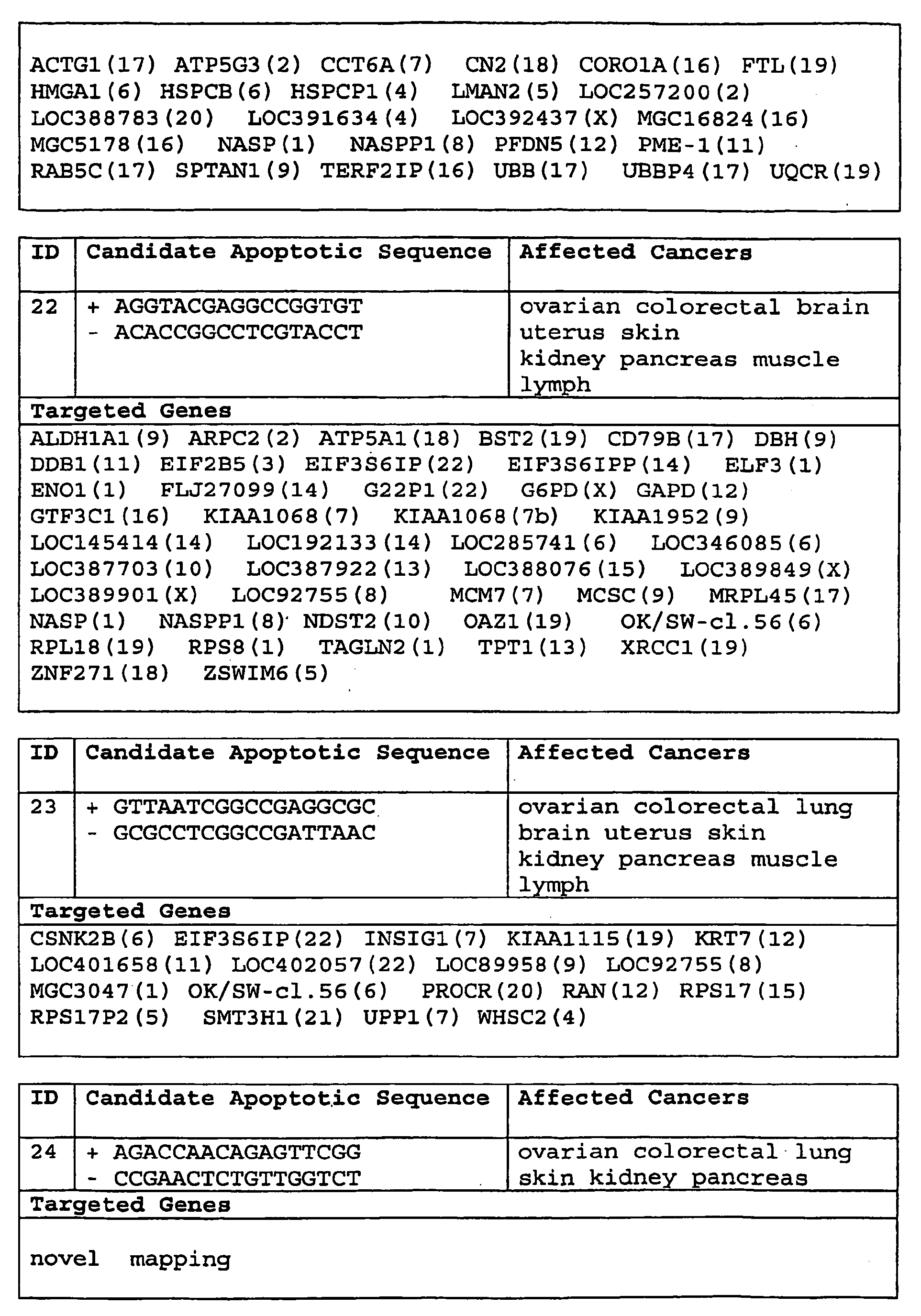

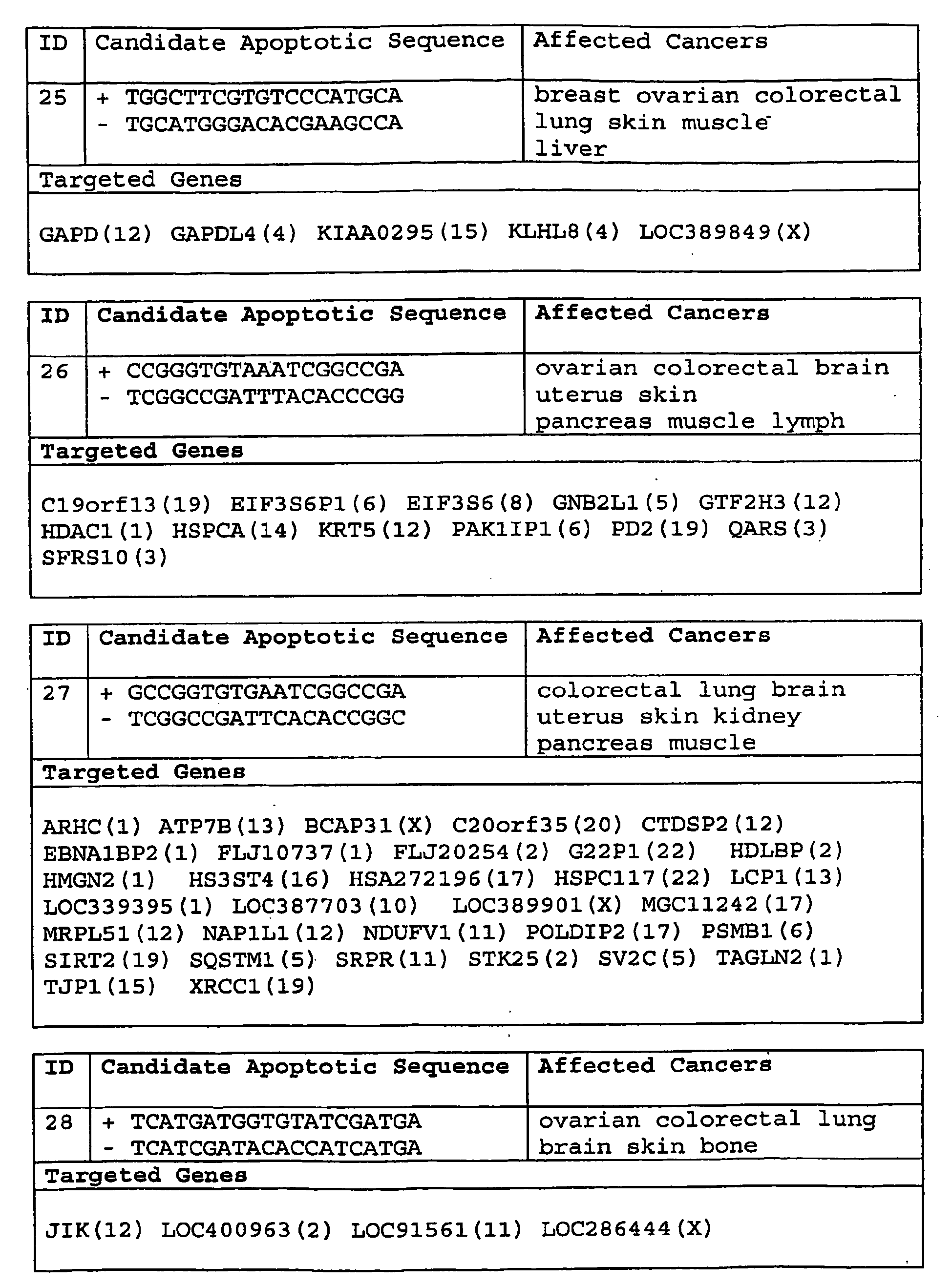

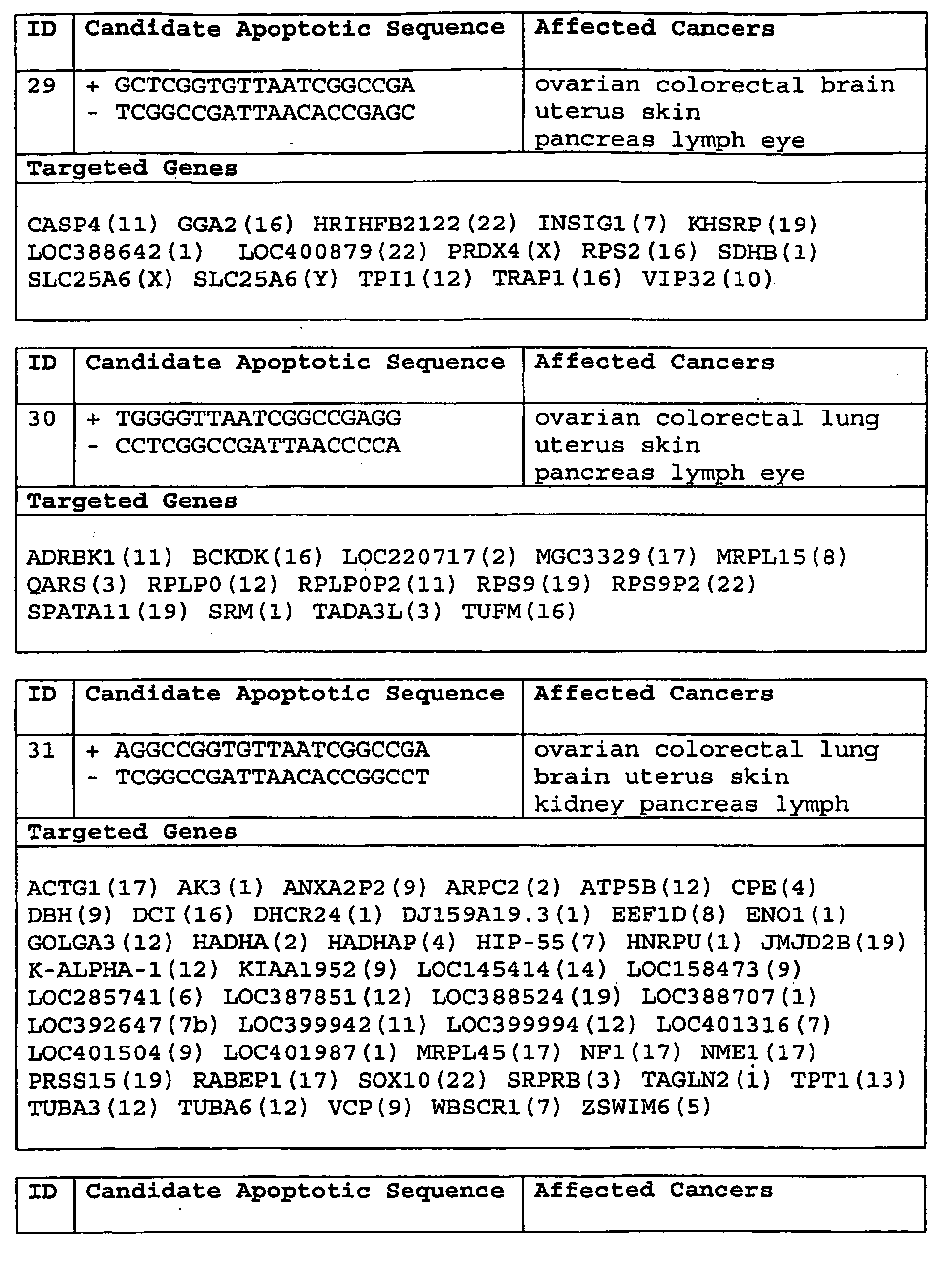

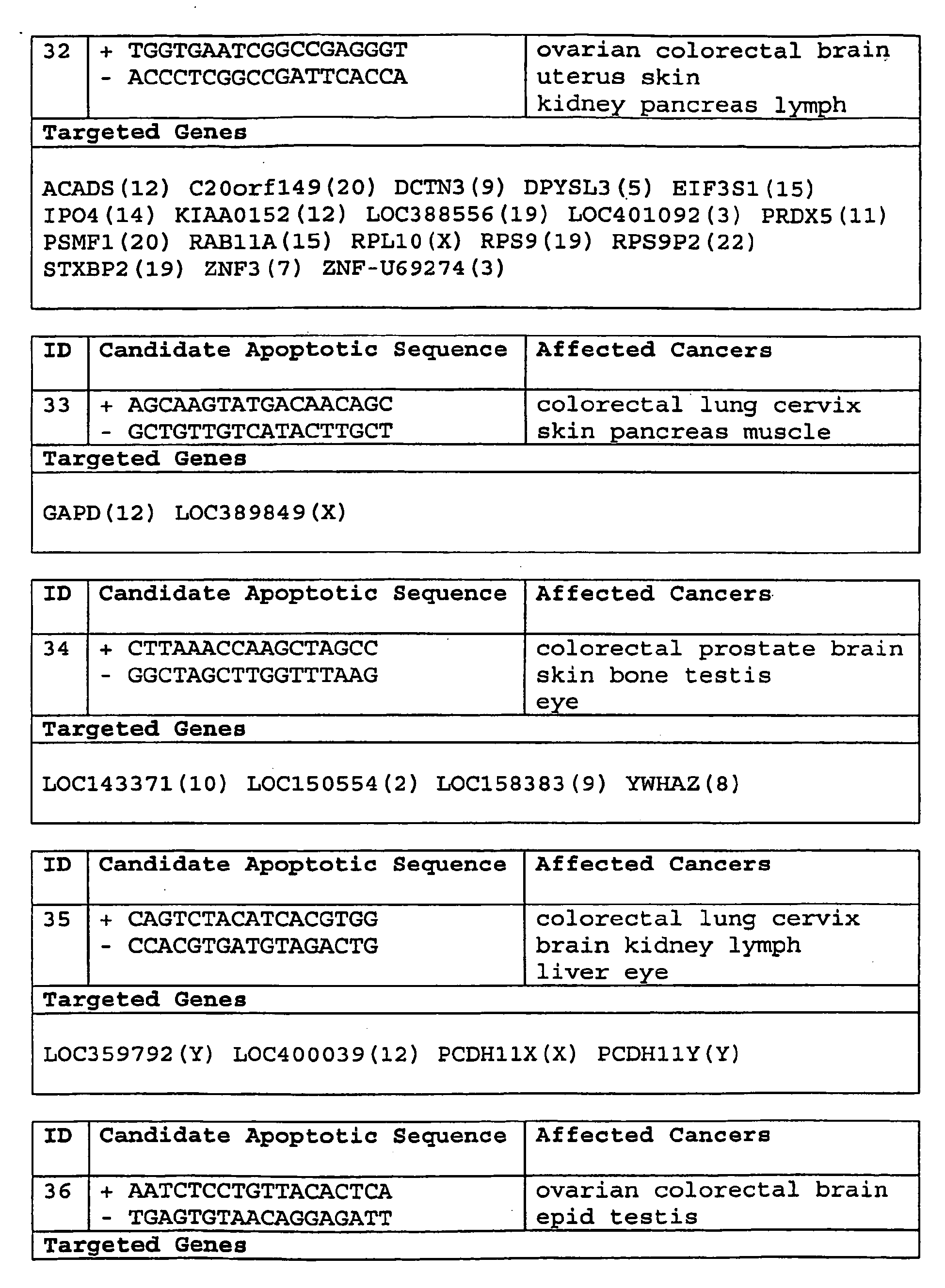

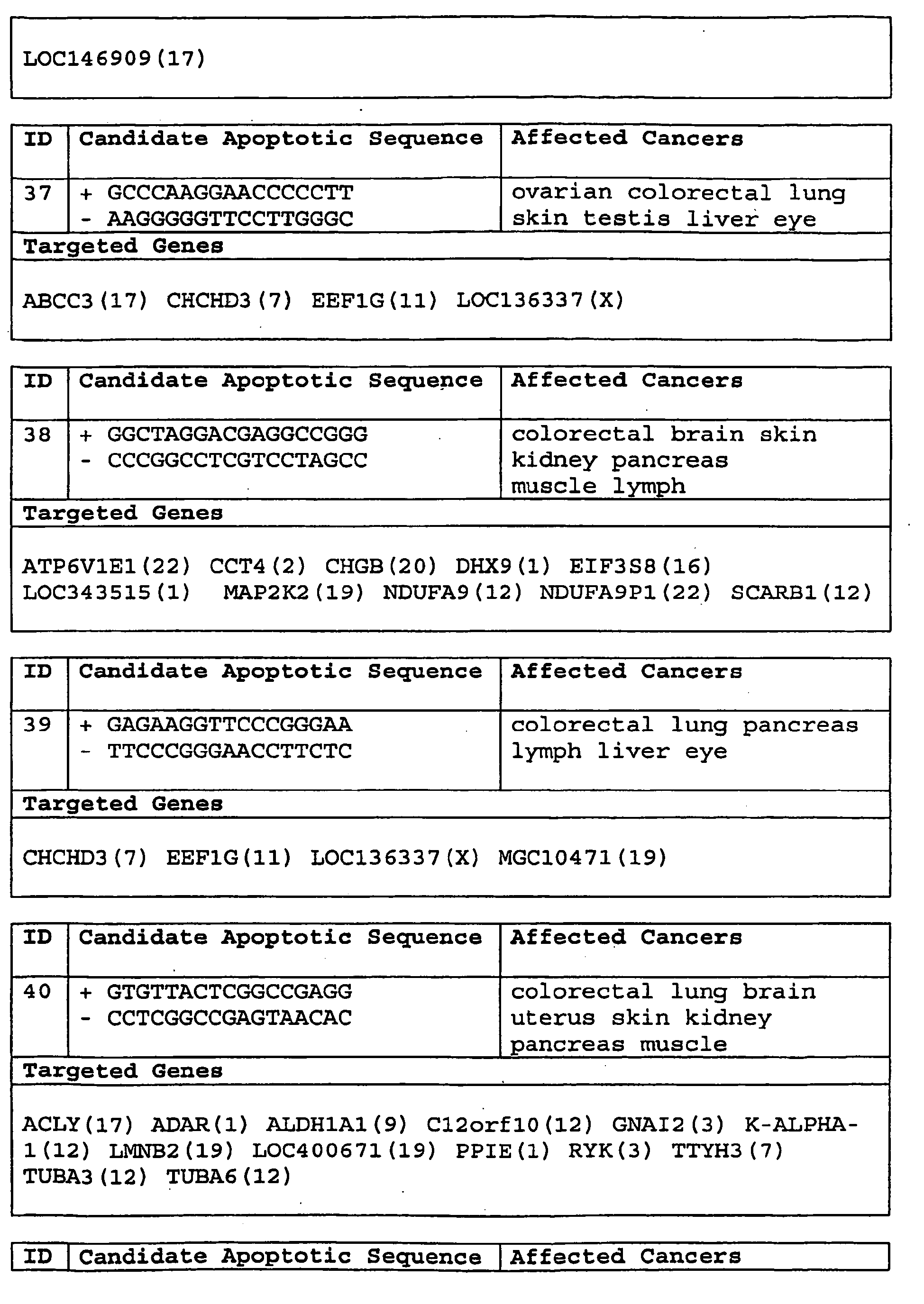

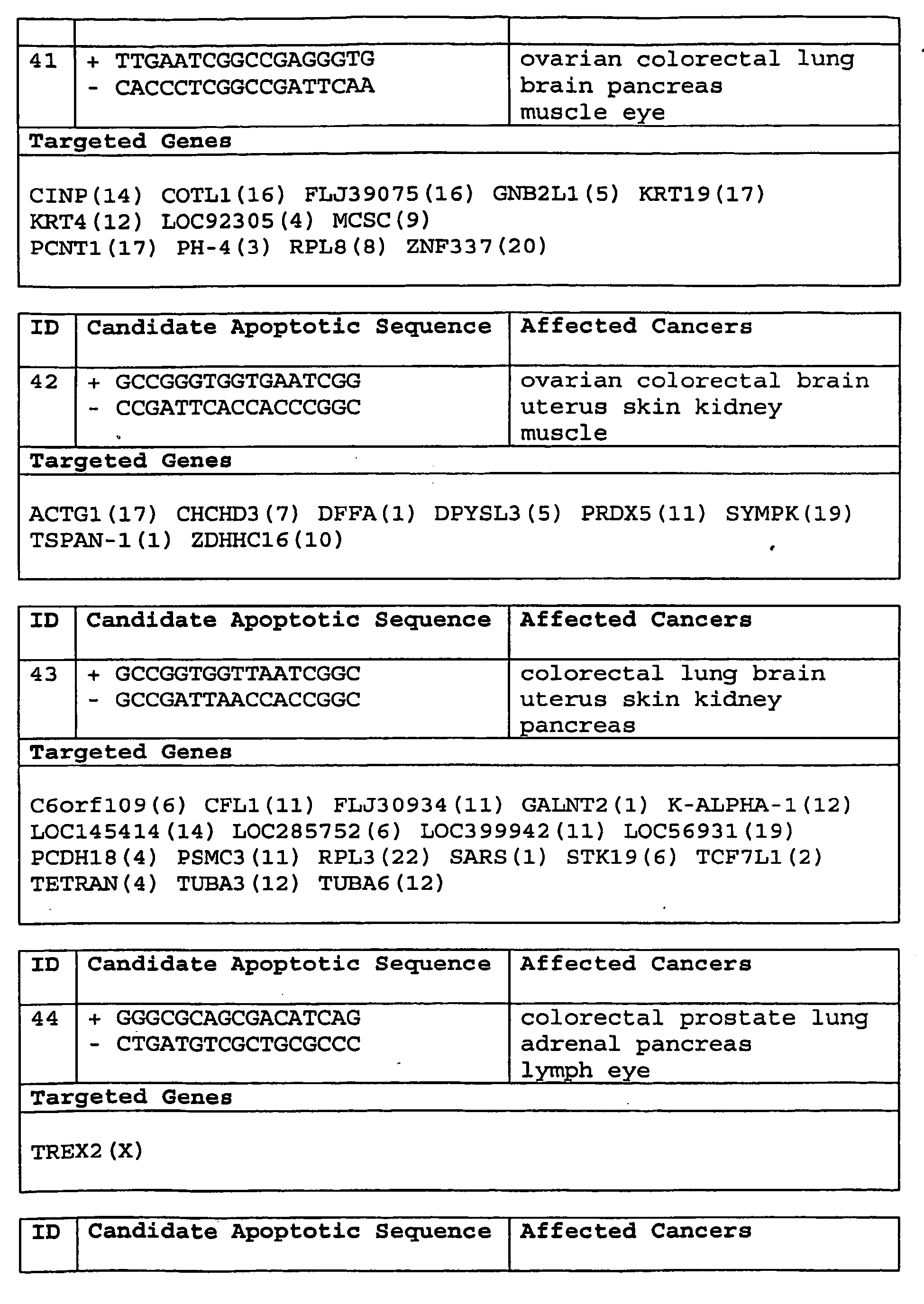

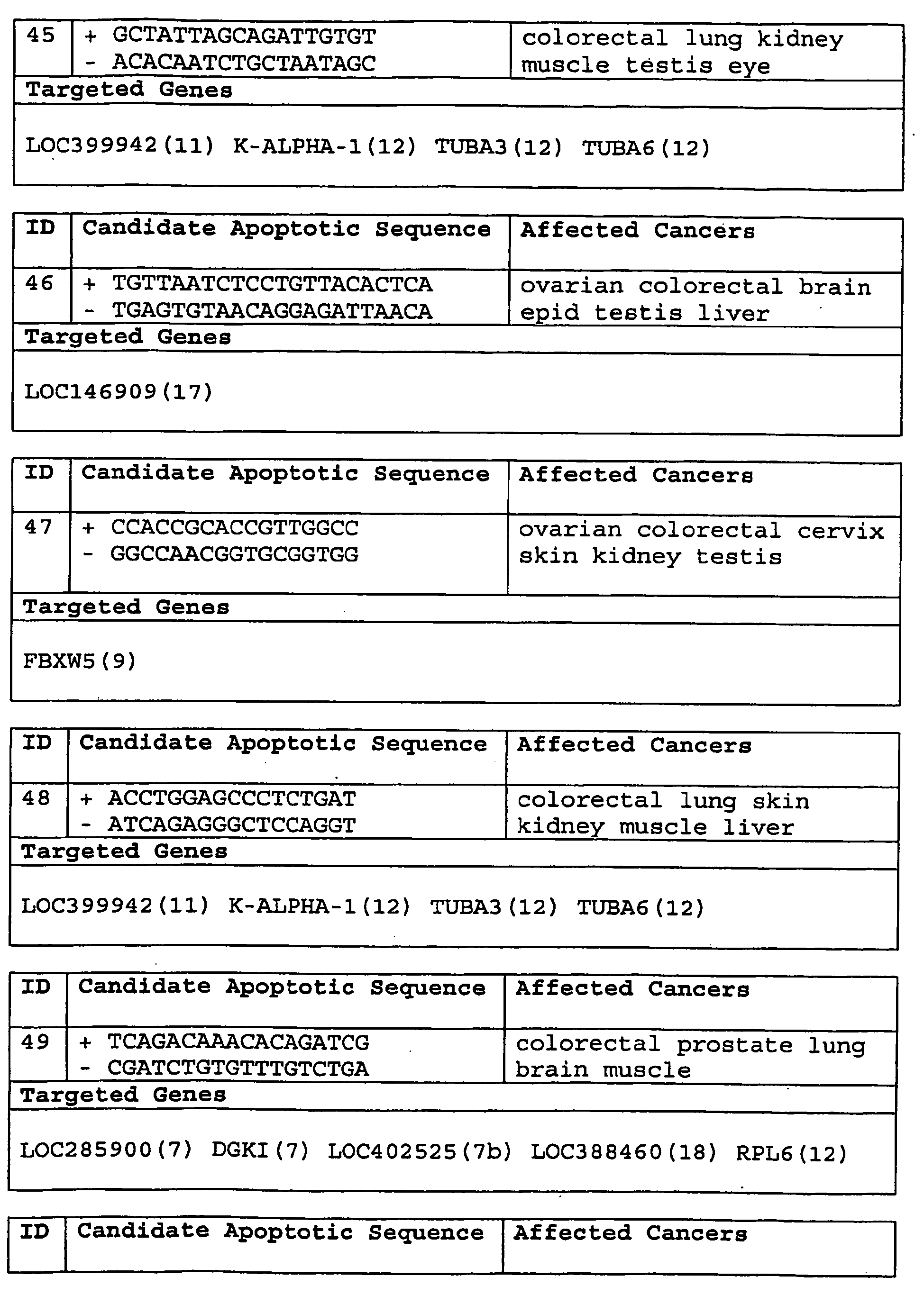

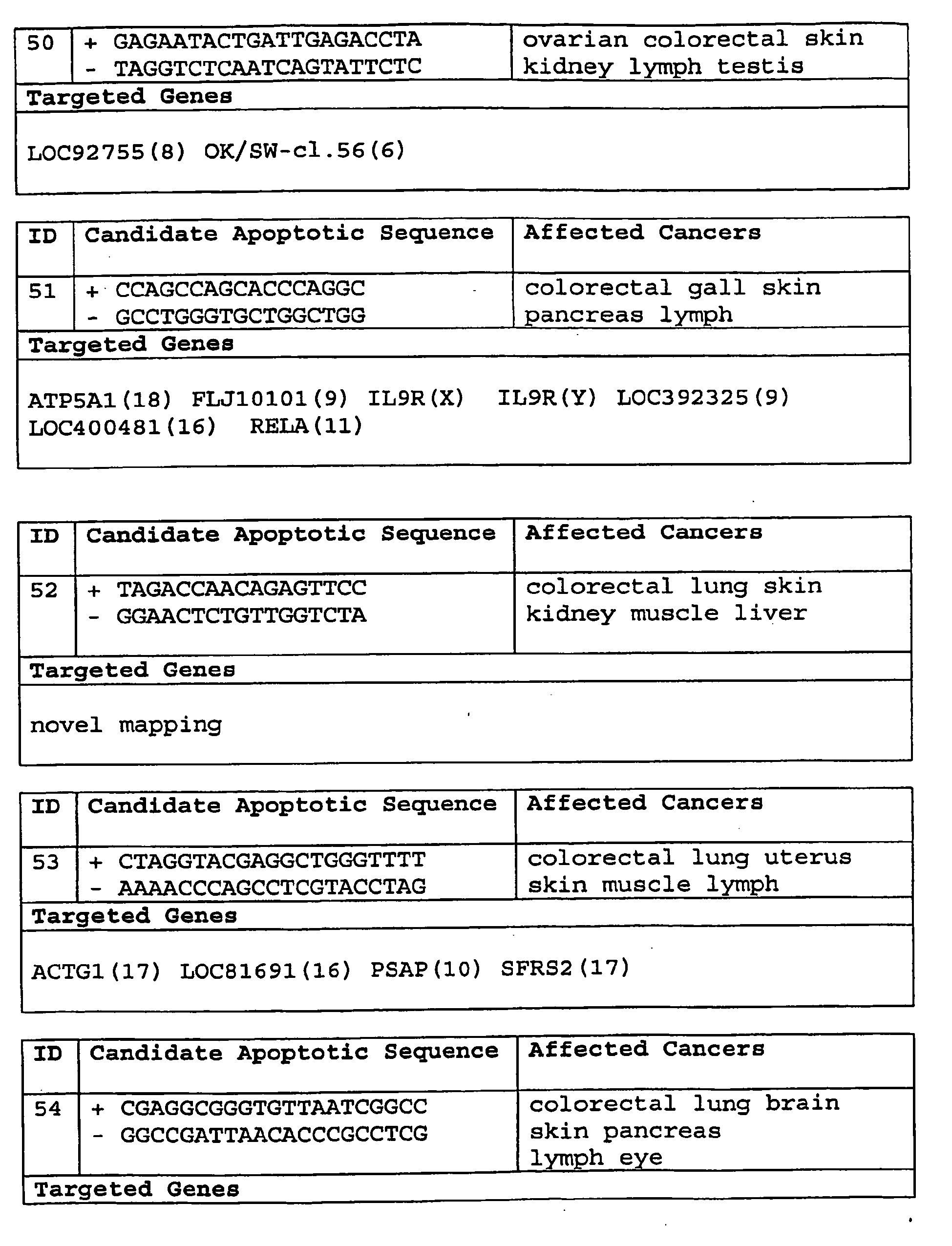

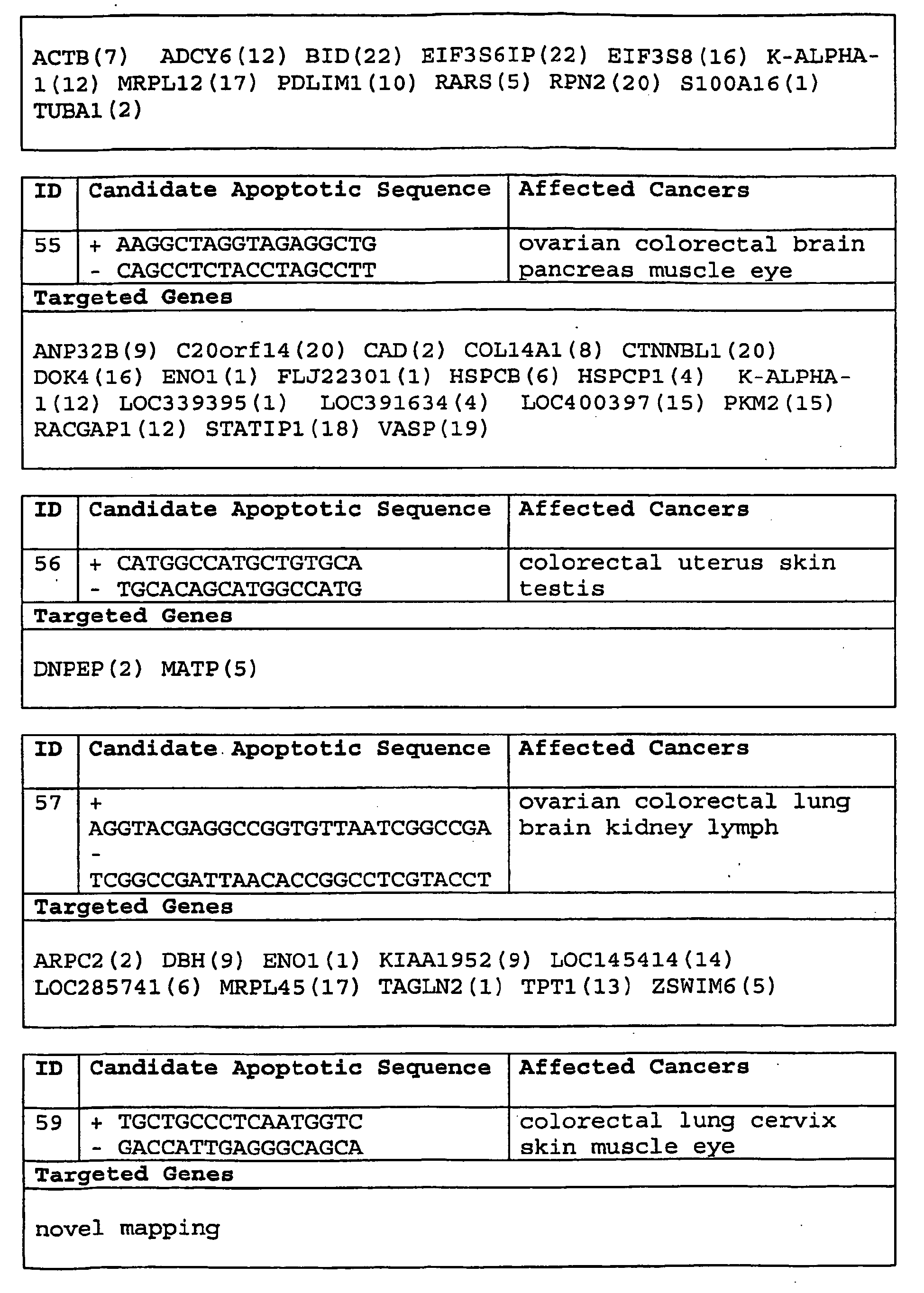

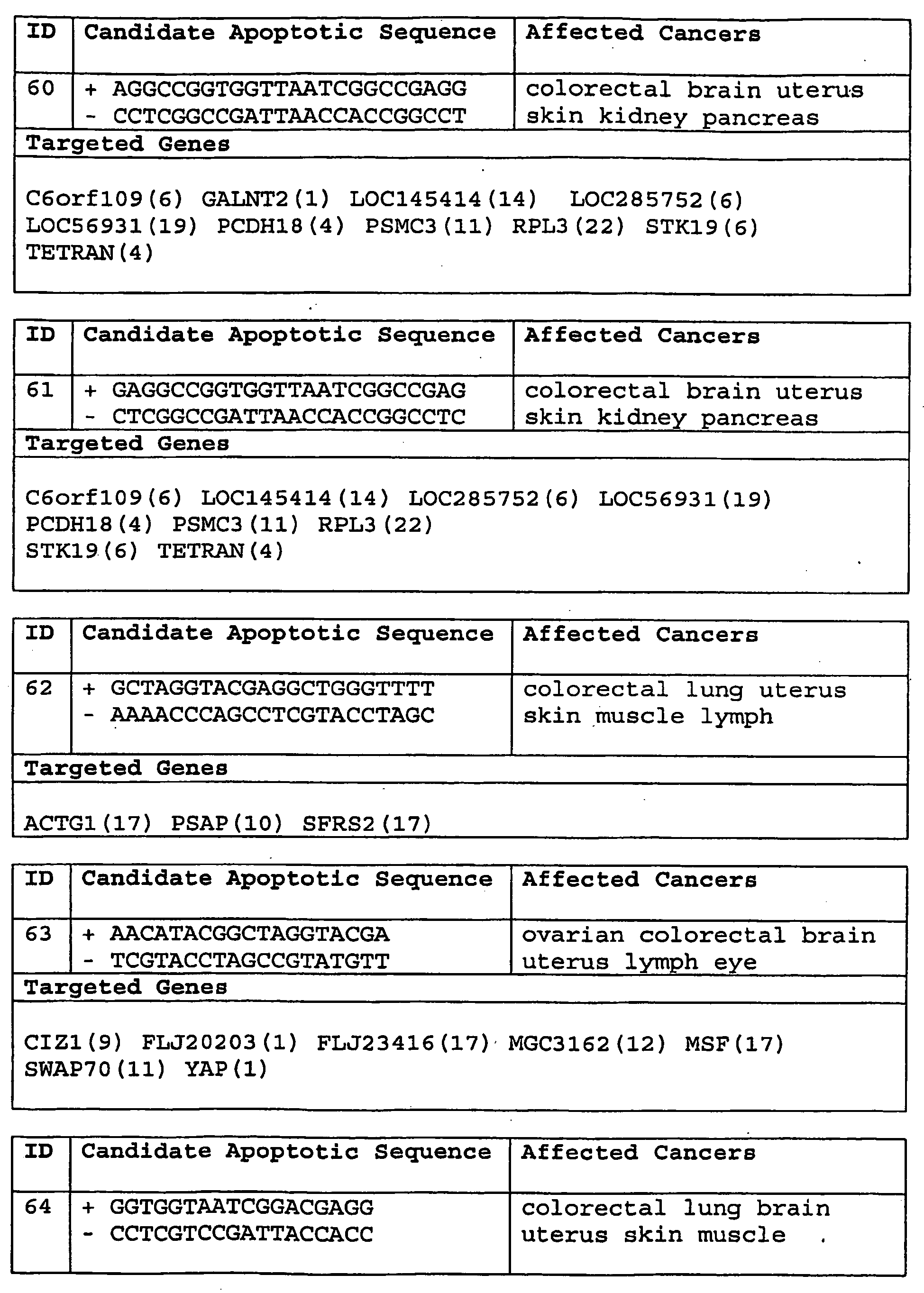

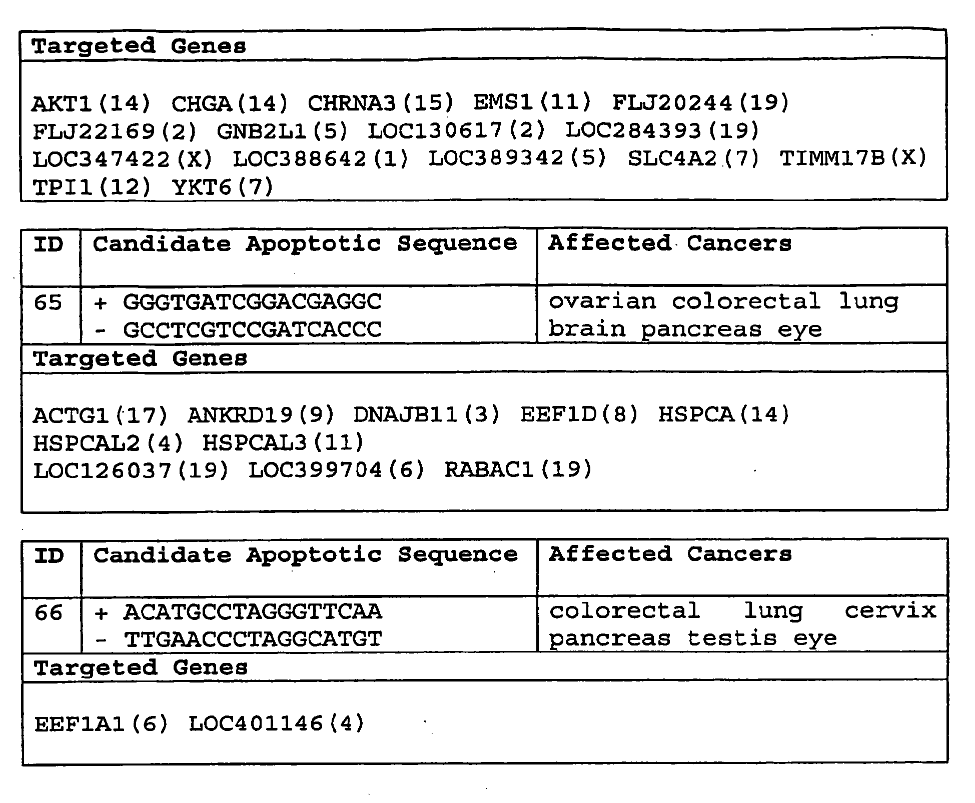

## Claims

1. A nucleic acid having a sequence selected from the group consisting of: Seq.ID.No:1, Seq.ID.No:2, Seq.ID.No:3, Seq.ID.No:4, Seq.ID.No: 5, Seq.ID.No:6, and Seq.ID.No:7.

2. A nucleic acid according to Claim 1, wherein the nucleic acid comprises DNA.

3. A nucleic acid according to Claim 2, wherein the nucleic acid comprises single stranded DNA.

4. A composition comprising:
a nucleic acid having a sequence selected from the group consisting of: Seq.ID.No:1, Seq.ID.No:2, Seq.ID.No:3, Seq.ID.No:4, Seq.ID.No: 5, Seq.ID.No:6, and Seq.ID.No:7; and
a pharmaceutically acceptable carrier.

5. A composition according to Claim 4, wherein the nucleic acid comprises DNA.

6. A composition according to claim 5, wherein the nucleic acid comprises single stranded DNA.

7. A composition according to Claim 4, comprising at least one additional nucleic acid having a sequence selected from the group consisting of: Seq.ID.No:1, Seq.ID.No:2, Seq.ID.No:3, Seq.ID.No:4, Seq.ID.No: 5, Seq.ID.No:6, and Seq.ID.No:7.

8. A composition according to Claim 4, further comprising a chemotherapeutic or radiotherapeutic.

9. A composition according to Claim 4, comprising a liquid carrier and the nucleic acid in a concentration of between about 0.17 mg/ml and 1.7 mg/ml.

10. A composition according to Claim 4, comprising the nucleic acid in a concentration of about 10 mg/ml.

11. A composition according to Claim 4, wherein the pharmaceutically acceptable carrier comprises phosphate buffered saline (PBS) or artificial cerebrospinal fluid (CSF).

12. Use of a nucleic acid having a sequence selected from the group consisting of: Seq. ID. No: 1, Seq. ID. No: 2, Seq. ID. No: 3, Seq. ID. No: 4, Seq. ID. No: 5, Seq. ID. No: 6 and Seq. ID. No: 7 for the manufacture of a medicament for the treatment of cancer.

13. Use according to claim 12, wherein the medicament further includes a second nucleic acid having the sequence selected from the group consisting of: Seq. ID. No: 2, Seq. ID. No: 3, Seq. ID. No: 4, Seq. ID. No: 5.

14. Use of a first nucleic acid having the sequence selected from the group consisting of: Seq. ID. No: 1, Seq. ID. No: 2, Seq. ID. No: 3, Seq. ID. No: 4, Seq. ID. No: 5, Seq. ID. No: 6, Seq. ID. No: 7 and a second nucleic acid having the sequence selected from the group consisting of: Seq. ID. No: 1, Seq. ID. No: 2, Seq. ID. No: 3, Seq. ID. No: 4, Seq. ID. No: 5, Seq. ID. No: 6 and Seq. ID. No: 7 for the manufacture of a medicament for separate administration for the treatment of cancer.

15. Use according to claim 12, wherein the medicament is suitable for use in a dose of between about 5 mg and 50 mg of nucleic acid per kg of subject.

16. Use according to claim 12, wherein the medicament is suitable for use in an amount effective to kill a cancer cell, but at a dose less than about 25 mg of nucleic acid per kg of subject.

17. Use according to claim 12, wherein the medicament is suitable for use weekly.

18. Use according to claim 12, wherein the medicament is suitable for use by intravenous drip.

19. Use of a nucleic acid having a sequence selected from the group consisting of: SEQ ID. No: 1, SEQ ID. No: 2, SEQ ID. No: 3, SEQ ID. No: 4, SEQ ID. No: 5, SEQ ID. No: 6 and SEQ ID. No: 7 for the in vitro detection of cancer cells.

## Patentansprüche

1. Nukleinsäure mit einer Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nur.5, Seq. ID. Nr. 6 und Seq. ID. Nr. 7.

2. Nukleinsäure nach Anspruch 1, wobei die Nukleinsäure DNA umfasst.

3. Nukleinsäure nach Anspruch 2, wobei die Nukleinsäure Einzelstrang-DNA umfasst.

4. Zusammensetzung, umfassend:
eine Nukleinsäure mit einer Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7; und
einen pharmazeutisch verträglichen Träger.

5. Zusammensetzung nach Anspruch 4, wobei die Nukleinsäure DNA umfasst.

6. Zusammensetzung nach Anspruch 5, wobei die Nukleinsäure Einzelstrang-DNA umfasst.

7. Zusammensetzung nach Anspruch 4, umfassend mindestens eine zusätzliche Nukleinsäure mit einer Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7.

8. Zusammensetzung nach Anspruch 4, ferner umfassend ein Chemotherapeutikum oder Radiotherapeutikum.

9. Zusammensetzung nach Anspruch 4, umfassend einen flüssigen Träger und die Nukleinsäure in einer Konzentration zwischen etwa 0,17 mg/ml und 1,7 mg/ml.

10. Zusammensetzung nach Anspruch 4, umfassend die Nukleinsäure in einer Konzentration von etwa 10 mg/ml.

11. Zusammensetzung nach Anspruch 4, wobei der pharmazeutisch verträgliche Träger phosphatgepufferte Kochsalzlösung (phosphate buffered saline; PBS) oder künstliche Zerebrospinalflüssigkeit (cerebrospinal fluid; CSF) umfasst.

12. Verwendung einer Nukleinsäure mit einer Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7 zur Herstellung eines Medikaments zur Behandlung von Krebs.

13. Verwendung nach Anspruch 12, wobei das Medikament ferner eine zweite Nukleinsäure mit der Sequenz, ausgewählt aus der Gruppe, bestehend aus Seq. ID. Nr. 1(siehe Ans. 14), Seq. ID. Nr. 2, Seq. ID. Nr. 3, Seq. ID. Nr.4, Seq. ID. Nr.5, Seq. ID. Nr. 6 und Seq. ID. Nr. 7(siehe Ans. 14), einschließt.

14. Verwendung einer ersten Nukleinsäure mit der Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7, und einer zweiten Nukleinsäure mit der Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7 zur Herstellung eines Medikaments zur getrennten Verabreichung zur Behandlung von Krebs.

15. Verwendung nach Anspruch 12, wobei das Medikament zur Verwendung in einer Dosis zwischen etwa 5 mg und 50 mg Nukleinsäure pro kg Proband geeignet ist.

16. Verwendung nach Anspruch 12, wobei das Medikament zur Verwendung in einer zum Abtöten einer Krebszelle wirksamen Menge, aber mit einer Dosis von weniger als etwa 25 mg Nukleinsäure pro kg Proband geeignet ist.

17. Verwendung nach Anspruch 12, wobei das Medikament zur wöchentlichen Verwendung geeignet ist.

18. Verwendung nach Anspruch 12, wobei das Medikament zur Verwendung durch intravenösen Tropf geeignet ist.

19. Verwendung einer Nukleinsäure mit einer Sequenz, ausgewählt aus der Gruppe, bestehend aus Seq. ID. Nr. 1, Seq. ID. Nr. 2, Seq. ID. Nr. 3, Seq. ID. Nr.4, Seq. ID. Nr.5, Seq. ID. Nr. 6 und Seq. ID. Nr. 7 zum in-vitro-Nachweis von Krebszellen

## Revendications

1. Un acide nucléique ayant une séquence choisie dans le groupe constitué par : SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7.

2. Un acide nucléique selon la revendication 1, où l'acide nucléique comprend de l'ADN.

3. Un acide nucléique selon la revendication 2, où l'acide nucléique comprend de l'ADN à brin unique.

4. Une composition comprenant :
un acide nucléique ayant une séquence choisie dans le groupe constitué par : SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7 ; et
un véhicule pharmaceutiquement acceptable.

5. Une composition selon la revendication 4, où l'acide nucléique comprend de l'ADN.

6. Une composition selon la revendication 5, où l'acide nucléique comprend de l'ADN à brin unique.

7. Une composition selon la revendication 4, comprenant au moins un acide nucléique additionnel ayant une séquence choisie dans le groupe constitué par : SEQ.ID.NO:1, SEQ.ID.NO:2, SEQ.ID.NO:3, SEQ.ID.NO:4, SEQ.ID.NO:5, SEQ.ID.NO:6 et SEQ.ID.NO:7.

8. Une composition selon la revendication 4, comprenant en outre un agent chimiothérapeutique ou radiothérapeutique.

9. Une composition selon la revendication 4, comprenant un véhicule liquide et l'acide nucléique à une concentration comprise entre environ 0,17 mg/ml et 1,7 mg/ml.

10. Une composition selon la revendication 4, comprenant l'acide nucléique à une concentration d'environ 10 mg/ml.

11. Une composition selon la revendication 4, où le véhicule pharmaceutiquement acceptable comprend une solution saline tamponnée phosphate (PBS) ou un fluide cérébrospinal artificiel (CSF).

12. Utilisation d'un acide nucléique ayant une séquence choisie dans le groupe constitué par: SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7 pour la fabrication d'un médicament pour le traitement du cancer.

13. Utilisation selon la revendication 12, où le médicament comprend en outre un second acide nucléique avec la séquence choisie dans le groupe constitué par : SEQ.ID.No:2, SEQ.ID.No:3, SEQ.ID.No:4, SEQ.ID.No:5.

14. Utilisation d'un premier acide nucléique avec la séquence choisie dans le groupe constitué par: SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7 et d'un second acide nucléique ayant une séquence choisie dans le groupe constitué par : SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7 pour la fabrication d'un médicament pour administration séparée pour le traitement du cancer.

15. Utilisation selon la revendication 12, où le médicament convient pour une utilisation à une dose comprise entre environ 5 mg et 50 mg d'acide nucléique par kg du patient.

16. Utilisation selon la revendication 12, où le médicament convient pour une utilisation en une quantité efficace pour tuer une cellule cancéreuse, mais à une dose inférieure à environ 25 mg d'acide nucléique par kg du patient.

17. Utilisation selon la revendication 12, où le médicament convient pour une utilisation hebdomadaire.

18. Utilisation selon la revendication 12, où le médicament convient pour une utilisation par goutte-à-goutte intraveineux.

19. Utilisation d'un acide nucléique ayant une séquence choisie dans le groupe formé par: SEQ.ID.No:1, SEQ.ID.NO:2, SEQ.ID.NO:3, SEQ.ID.NO:4, SEQ.ID.NO:5, SEQ.ID.NO:6 et SEQ.ID.NO:7 pour la détection *in vitro* de cellules cancéreuses.
